(19) Europäisches Patentamt
European Patent Office
Office européen des brevets

(11) EP 4 065 235 B1

(12) **EUROPÄISCHE PATENTSCHRIFT**

(45) Veröffentlichungstag und Bekanntmachung des Hinweises auf die Patenterteilung:
**21.01.2026 Patentblatt 2026/04**

(21) Anmeldenummer: **20774946.6**

(22) Anmeldetag: **16.09.2020**

(51) Internationale Patentklassifikation (IPC):
***A61Q 5/06*** *(2006.01)* ***A61K 8/73*** *(2006.01)*
***A61K 8/898*** *(2006.01)*

(52) Gemeinsame Patentklassifikation (CPC):
**A61Q 5/065; A61K 8/73; A61K 8/731; A61K 8/732; A61K 8/733; A61K 8/898;** A61K 2800/30

(86) Internationale Anmeldenummer:
**PCT/EP2020/075880**

(87) Internationale Veröffentlichungsnummer:
**WO 2021/104706 (03.06.2021 Gazette 2021/22)**

# (54) MITTEL ZUM FÄRBEN VON KERATINISCHEM MATERIAL MIT AMINOSILIKON, PIGMENT UND POLYSACCHARID

AGENT FOR DYEING KERATINOUS MATERIAL, CONTAINING AMINOSILICONE, PIGMENT AND POLYSACCHARIDE

AGENT POUR COLORER DES MATIÈRES KÉRATINIQUES, CONTENANT DE L'AMINOSILICONE, UN PIGMENT ET UN POLYSACCHARIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.11.2019 DE 102019218234**

(43) Veröffentlichungstag der Anmeldung:
**05.10.2022 Patentblatt 2022/40**

(73) Patentinhaber: **Henkel AG & Co. KGaA**
**40589 Düsseldorf (DE)**

(72) Erfinder:
- **KRUCK, Constanze**
  **41515 Grevenbroich (DE)**
- **HILBIG, Sandra**
  **44894 Bochum (DE)**
- **MOCH, Melanie**
  **41542 Dormagen (DE)**
- **DICKHOF, Susanne**
  **41748 Viersen (DE)**
- **KESSLER-BECKER, Daniela**
  **51371 Leverkusen (DE)**

(56) Entgegenhaltungen:
**WO-A1-2011/128255**
**DE-A1- 102013 226 102**
**FR-A1- 2 944 967**
**US-A1- 2011 104 094**
**US-A1- 2016 051 023**

- **DATABASE GNPD [online] MINTEL; 9 November 2017 (2017-11-09), ANONYMOUS: "Hair Spray", XP055749937, retrieved from www.gnpd.com Database accession no. 5238685**
- **UDO PEETZ: "CREATING TOMORROW'S SOLUTIONS Wacker-Belsil ? ADM Grades", 1 January 2004 (2004-01-01), pages 1 - 25, XP055206796, Retrieved from the Internet <URL:-> [retrieved on 20150807]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 4 065 235 B1

## Beschreibung

Gegenstand der vorliegenden Anmeldung ist ein Mittel zum Färben von keratinischem Material, insbesondere menschlichen Haaren, welches mindestens ein lineares, aminofunktionalisiertes Silikonpolymer (a1), mindestens ein Pigment (a2) mit einer Löslichkeit in Wasser bei 25 °C von weniger als 0,05 g/L, und mindestens ein Polysaccharid (a3), das ausgewählt ist aus der Gruppe aus Hydroxy-$C_1$-$C_{30}$-alkylcellulosen, Hydroxy-$C_1$-$C_{30}$-alkylmethylcellulosen, Hydroxy-$C_1$-$C_{30}$-alkylethylcellulosen, $C_1$-$C_{30}$-Alkylcellulosen, $C_6$-$C_{30}$-Alkylhydroxyethylcellulosen, Cellulose, Stärke, Xanthan, Carboxyalkylcellulose, Alginsäure, Carrageen, Tragant, Karaya-Gummi, Gummi Arabicum, Gellan, Pektin, Agaropektin und/oder deren Salzen, enthält. Des weiteren ist das Mittel dadurch gekennzeichnet, dass es frei ist von Silikonharzen.

Ein zweiter Gegenstand dieser Anmeldung ist ein Verfahren zum Färben von keratinischem Material, insbesondere menschlichen Haaren, wobei ein Mittel des ersten Erfindungsgegenstands auf dem keratinischen Material angewendet, einwirken gelassen und danach wieder mit Wasser ausgewaschen wird.

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

US 2011/0104094 A1 adressiert Konditioniermittel mit kationischen Tensiden, Fettstoffen, direktziehenden Farbstoffen und nichtionischen Verdicker-Polymeren.

US 2016/051023 A1 beschreibt ein Verfahren zum Färben und semipermanenten Glätten von Haaren.

In DE 10 2013 226102 A1 werden wasserhaltige Färbemittel für Keratinfasern mit einem pH-Wert von 1,0 bis 5,5 beschrieben, die ein Aminosilikon einer Formel (I) und mindestens einen direktziehenden Säurefarbstoff enthalten.

In der Datenbank Mintel wird das in Spayform vorliegende Marktprodukt "Retok Hair Spray" (XP055749937, Datenbank-Nummer 5238685) offenbart, welches ein lineares Aminosilikon, einen direktziehenden Säurefarbstoff sowie Xanthan als Polysaccharid enthält.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren. Ein mögliches, alternatives Färbesystem, das in letzter Zeit zunehmend in den Fokus rückt, beruht auf dem Einsatz von farbigen Pigmenten.

Die Färbung mit Pigmenten bietet verschiedene wesentliche Vorteile. Da die Pigmente sich lediglich von außen an die Keratinmaterialen, insbesondere an die Haarfasern, anlagern, ist die mit dem Färbeprozess verbundene Schädigung ganz besonders gering. Weiterhin lassen sich nicht mehr erwünschte Färbungen schnell und einfach rückstandslos entfernen und bieten dem Anwender auf diese Weise die Möglichkeit, unmittelbar und ohne großen Aufwand zu seiner Ursprungshaarfarbe zurückkehren zu können. Insbesondere für die Konsumenten, die ihre Haare nicht regelmäßig nachfärben möchten, ist dieser Färbeprozess daher besonders attraktiv.

In aktuellen Arbeiten wurde das Problem der geringen Haltbarkeit dieses Färbesystems adressiert. In diesem Zusammenhang konnte gefunden werden, dass die Waschechtheit der mit Pigmenten erhaltenen Farbresultate durch Kombination der Pigmente mit bestimmten aminofunktionalisierten Silikonpolymeren stark verbessert werden konnte. Trotz der so gefundenen Möglichkeiten, die Waschechtheit bzw. den Farberhalt zu verbessern, besteht in diesem Zusammenhang jedoch immer noch Optimierungsbedarf. Besonders für auffällige und modische Nuancen wird nach einem Weg gesucht, die Farbintensität noch weiter zu verbessern.

Auf Pigmenten basierende Färbesysteme werden beispielsweise in EP 3058935 B1 beschrieben. Zur Verbesserung der Abscheidung von Pigmenten auf den Haaren und für die Erzeugung von natürlich wirkenden Färbungen

schlägt EP 3058935 B1 die Anwendung einer Formulierung vor, die in einer wässrig-alkoholischen Basis neben einem Pigment weiterhin ein Aminosilikon, ein Silikon-harz und ein Verdickersystem aus verschiedenen Polymeren enthält.

Bei der Nacharbeitung der Lehre der EP 3058935 B1 hat sich nun gezeigt, dass die im Mittel enthaltenen Silikonharze auch einen nachteiligen Einfluss auf die gefärbten Haare ausüben können. Zum einen hat sich gezeigt, dass die Silikonharze das Griffgefühlt der gefärbten Haare signifikant verschlechtern. Darüber hinaus hat sich ebenfalls herausgestellt, dass die entsprechenden Färbemittel mit Silikonharzen auch mit Blick auf die Farbintensität deutlich unterlegen sind.

Es war die Aufgabe der vorliegenden Erfindung, ein Färbesystem bereitzustellen, mit dem Pigmente sich in möglichst dauerhafter Weise auf den Haaren fixieren lassen. Mit der Anwendung dieses Färbesystems sollten besonders intensive Färbeergebnisse mit guten Echtheitseigenschaften erzielt werden. Gleichzeitig sollten die Haare ein möglichst angenehmes Griffgefühl aufweisen und sich weder fettig noch beschwert, stumpf oder belegt anfühlen.

Überraschenderweise hat sich nun herausgestellt, dass die vorgenannte Aufgabe hervorragend gelöst werden kann, wenn keratinische Materialien, insbesondere Haare, mit einem Mittel gefärbt werden, welches mindestens ein lineares aminofunktionalisiertes Silikonpolymer (a1), mindestens Pigment (a2) und mindetens ein Polysaccharid (a3) enthalten. Weiterhin hat sich in Versuchsreihen herausgestellt, dass insbesondere dann gute Färbeeffekte erhalten wurde, wenn die entsprechenden Mittel frei von Silikonharzen waren.

Ein erster Gegenstand der vorliegenden Erfindung ist ein Mittel zum Färben von keratinischem Material, insbesondere menschlichen Haaren, enthaltend

(a1) mindestens ein lineares, aminofunktionalisiertes Silikonpolymer, und
(a2) mindestens ein Pigment mit einer Löslichkeit in Wasser bei 25 °C von weniger als 0,05 g/L, und
(a3) mindestens ein Polysaccharid, das ausgewählt ist aus der Gruppe aus Hydroxy-$C_1$-$C_{30}$-alkylcellulosen, Hydroxy-$C_1$-$C_{30}$-alkylmethylcellulosen, Hydroxy-$C_1$-$C_{30}$-alkylethyl-cellulosen, $C_1$-$C_{30}$-Alkylcellulosen, $C_6$-$C_{30}$-Alkylhydroxyethylcellulosen, Cellulose, Stärke, Xanthan, Carboxyalkylcellulose, Alginsäure, Carrageen, Tragant, Karaya-Gummi, Gummi Arabicum, Gellan, Pektin, Agaropektin und/oder deren Salzen,

dadurch gekennzeichnet, dass das Mittel frei ist von Silikonharzen

Im Zuge der zu dieser Erfindung durchgeführten Arbeiten hat sich überraschenderweise gezeigt, dass der Einsatz eines Polysaccharids (a3) in einem Mittel, welches ein Aminosilikon (a1), und ein Pigment (a2) und einen Fettbestandteil (a4) enthält, zu einer Erhöhung der Farbintensität führt, wenn dieses Mittel in einem Färbeverfahren auf dem keratinschen Material, insbesondere auf menschlichen Haaren, angewendet wird. Hierbei musste das mittel frei von Silkonharzen sein.

keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Mittel zur Färbung

Der Begriff "Mittel zur Färbung" wird im Rahmen dieser Erfindung für eine durch Einsatz von farbgebenden Verbindungen, insbesondere Pigmenten, hervorgerufene Farbgebung des Keratinmaterials, insbesondere des Haares, verwendet. Bei dieser Färbung lagern sich die Pigmente als farbgebende Verbindungen in einem besonders homogenen, gleichmäßigen und glatten Film an der Oberfläche des Keratinmaterials ab.

Lineare aminofunktionalisierte Silikonpolymere (a1)

Als ersten erfindungswesentlichen Inhaltsstoff (a1) enthält das Mittel mindestens ein lineares aminofunktionalisiertes Silikonpolymer. Das aminofunktionalisiertes Silikonpolymer kann alternativ auch als Aminosilikon oder Amodimethicone bezeichnet werden.

Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.

Das maximale Molekulargewicht des Silikonpolymers hängt von dem Polymerisationsgrad (Anzahl der polymerisierten Monomere) und der Ansatzgröße ab und wird durch die Polymerisationsmethode mitbestimmt. Im Sinne der

vorliegenden Erfindung ist es bevorzugt, wenn das maximale Molekulargewicht des Silikonpolymers nicht mehr als $10^7$ g/mol, bevorzugt nicht mehr als $10^6$ g/mol und besonders bevorzugt nicht mehr als $10^5$ g/mol beträgt.

Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bezeichnet.

In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.

Unter einem aminofunktionalisierten Silikonpolymer wird ein funktionalisiertes Silikon verstanden, welches mindestens eine Struktureinheit mit einer Aminogruppe trägt. Bevorzugt trägt das aminofunktionalisierte Silikonpolymer mehrere Struktureinheiten mit jeweils mindestens einer Aminogruppe. Unter einer Aminogruppe wird eine primäre Aminogruppe, eine sekundäre Aminogruppe und eine tertiäre Aminogruppe verstanden. Alle diese Aminogruppen können im sauren Milieu protoniert werden und liegen dann in ihrer kationischen Form vor.

Unter einem linearen Silkonpolymerwird lineares Polymer ohne Verzweigungen verstanden, das nur aus einer Polymerkette, der Hauptkette, besteht.

Mit anderen Worten ist ein lineares aminofunktionalisiertes Silikonpolymer ein Polysiloxan des Typs (Si-linear)

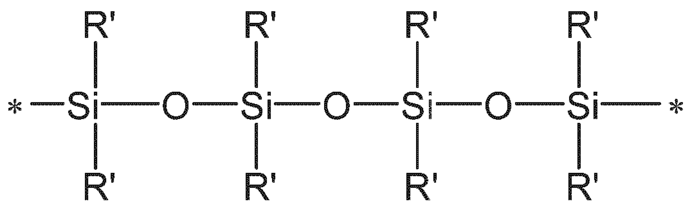

(Si-Linear),

d.h. die an den Siliciumatomen befindlichen Reste R' können zwar (jeweils unabhängig voneinander) organische Reste wie Methylgruppen, $C_1$-$C_{20}$-Alkylgruppen, Amino-$C_1$-$C_{20}$ alkylgruppen oder substituierte $C_1$-$C_{20}$-Alkylgruppen darstellen, aber die durch die Abfolge von Siliciumatomen und Saurestoffatomen gebildete Siloxan-Hauptkette selbst ist nicht verzeigt und somit linear.

Prinzipiell konnten gute Effekte mit linearen aminofunktionalisierten Silikonpolymeren (a1) erzielt werden, wenn diese mindestens eine primäre, mindestens eine sekundäre und/oder mindestens eine tertiäre Aminogruppe tragen. Färbungen mit der besten Waschechtheit wurden jedoch beobachtet, wenn ein lineares aminofunktionalisiertes Silikonpolymer (a1) im Mittel (a) eingesetzt wurde, welches mindestens eine sekundäre Aminogruppe enthält.

In einer ganz besonders bevorzugten Ausführungsform ist ein erfindungsgemäßes Verfahren dadurch gekennzeichnet, dass das Mittel (a) mindestens ein lineares aminofunktionalisiertes Silikonpolymer (a1) mit mindestens eine sekundären Aminogruppe enthält.

Die sekundäre Aminogruppe(n) kann bzw. können sich an verschiedenen Positionen des aminofunktionalisierten Silikonpolymers befinden. Ganz besonders gute Effekt wurden gefunden, wenn ein aminofunktionalisiertes Silikonpolymer (a1) eingesetzt wurde, dass mindestens eine, bevorzugt mehrere Struktureinheiten der Formel (Si-Amino) besitzt.

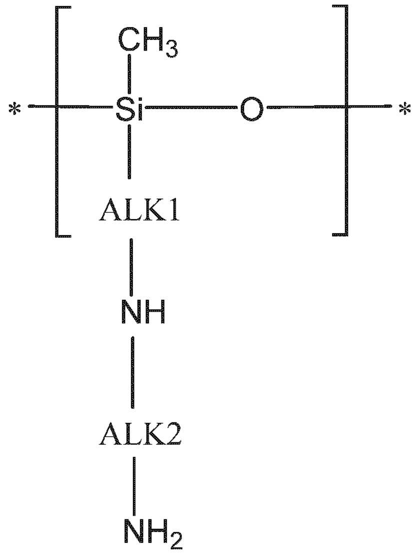

(Si-Amino)

In den Struktureinheiten der Formel (Si-Amino) steht die Kürzel ALK1 und ALK2 unabhängig voneinander für eine lineare oder verzweigte, zweiwertige $C_1$-$C_{20}$-Alkylengruppe.

In einer weiteren ganz besonders bevorzugten Ausführungsform ist ein erfindungsgemäßes Verfahren dadurch gekennzeichnet, dass das Mittel (a) mindestens ein lineares aminofunktionalisiertes Silikonpolymer (a1) enthält, das mindestens eine Struktureinheit der Formel (Si-Amino) umfasst,

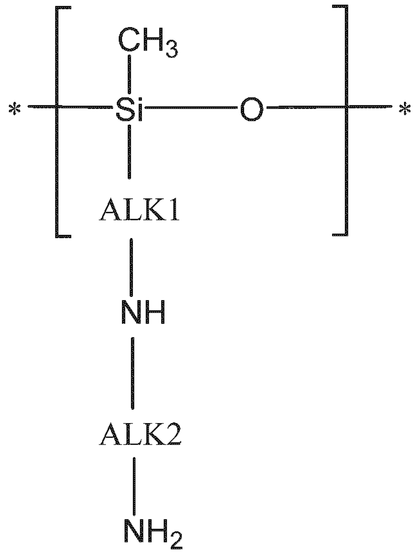

(Si-Amino)

wobei

ALK1 und ALK2 unabhängig voneinander für eine lineare oder verzweigte, zweiwertige $C_1$-$C_{20}$-Alkylengruppe stehen.

Die mit einem Stern (*) gekennzeichneten Position geben hierbei jeweils die Bindung zu weiteren Struktureinheiten des Silikonpolymers an. Beispielsweise kann das dem Stern benachbarte Silicium-Atom an ein weiteres Sauerstoffatom gebunden sein, und das dem Stern benachbarte Sauerstoffatom kann an ein weiteres Siliciumatom oder auch an eine $C_1$-$C_6$-Alkylgruppe gebunden sein.

Eine zweiwertige $C_1$-$C_{20}$-Alkylengruppe kann alternativ auch als eine divalente oder zweibindige $C_1$-$C_{20}$-Alkylengruppe bezeichnet werden, womit gemeint ist, dass jede Gruppierung ALK1 bzw. AK2 zwei Bindungen eingehen kann.

Im Fall von ALK1 erfolgt eine Bindung vom Silicium-Atom zur Gruppierung ALK1, und die zweite Bindung besteht zwischen ALK1 und der sekundären Aminogruppe.

Im Fall von ALK2 erfolgt eine Bindung von der sekundären Aminogruppe zur Gruppierung ALK2, und die zweite

Bindung besteht zwischen ALK2 und der primären Aminogruppe.

Beispiele für eine lineare zweiwertige $C_1$-$C_{20}$-Alkylengruppe sind beispielsweise die Methylen-gruppe ($-CH_2-$), die Ethylengruppe ($-CH_2-CH_2-$), die Propylengruppe ($-CH_2-CH_2-CH_2-$) und die Butylengruppe ($-CH_2-CH_2-CH_2-CH_2-$). Die Propylengruppe ($-CH_2-CH_2-CH_2-$) ist besonders bevorzugt. Ab einer Kettenlänge von 3 C-Atomen können zweiwertige Alkylengruppen auch verzweigt sein. Beispiele für verzweigte, zweiwertige $C_3$-$C_{20}$-Alkylengruppen sind ($-CH_2-CH(CH_3)-$) und ($-CH_2-CH(CH_3)-CH_2-$).

In einer weiteren besonders bevorzugten Ausführungsform stellen die Struktureinheiten der Formel (Si-Amino) Wiederholungseinheiten im lienaren aminofunktionalisierten Silikonpolymer (a1) dar, so dass das Silikonpolymer mehrer Struktureinheiten der Formel (Si-Amino) umfasst.

Im Folgenden werden besonders gut geeignete aminofunktionalisierte Silikonpolymere (a1) mit mindestens einer sekundären Aminogruppe aufgelistet.

Färbungen mit den allerbesten Waschechtheiten konnten erhalten werden, wenn im erfindungsgemäßen Verfahren mindestens ein Mittel (a) auf dem keratinischen Material appliziert wurde, das mindestens ein lineares aminofunktionalisiertes Silikonpolymer (a1) enthält, das Struktureinheiten der Formel (Si-I) und der Formel (Si-II) umfasst

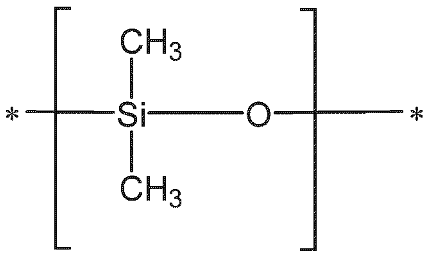

(Si-I)

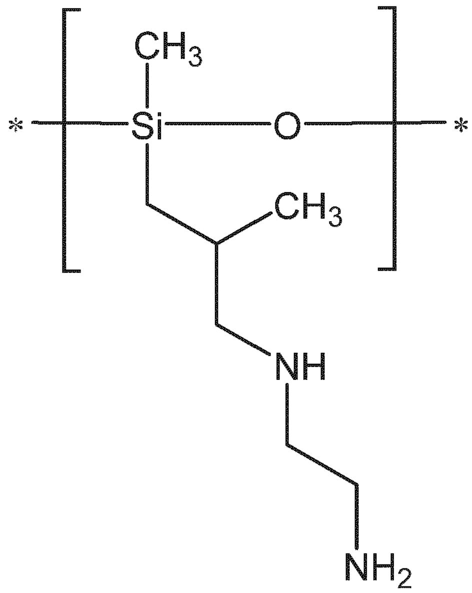

(Si-II).

In einer weiteren explizit ganz besonders bevorzugten Ausführungsform ist ein erfindungsgemäßes Verfahren dadurch gekennzeichnet, dass das Mittel (a) mindestens ein lineares aminofunktionalisiertes Silikonpolymer (a1) enthält, das Struktureinheiten der Formel (Si-I) und der Formel (Si-II) umfasst

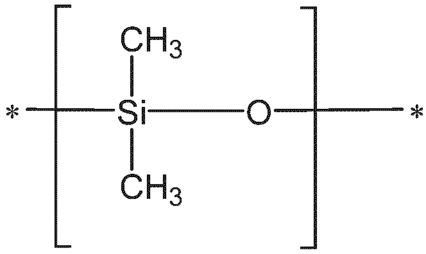

(Si-I)

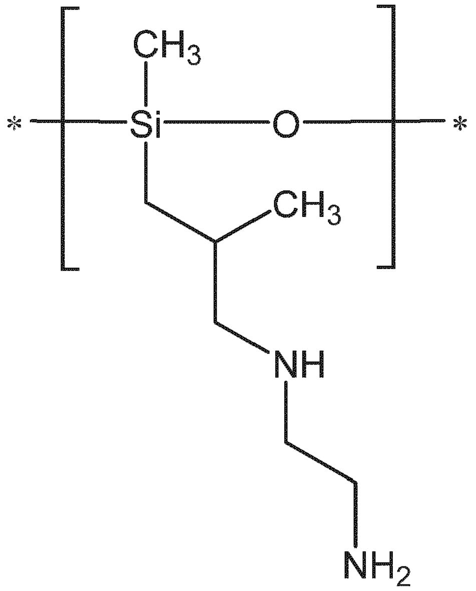

(Si-II).

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt.

Im Rahmen einer weiteren bevorzugten Ausführungsform ist ein erfindungsgemäßes Verfahren gekennzeichnet durch die Anwendung eines Mittels (a) auf dem keratinischem Material, wobei das Mittel (a) mindestens ein aminofunktionelles Silikonpolymer (a1) der Formel der Formel (Si-III) enthält,

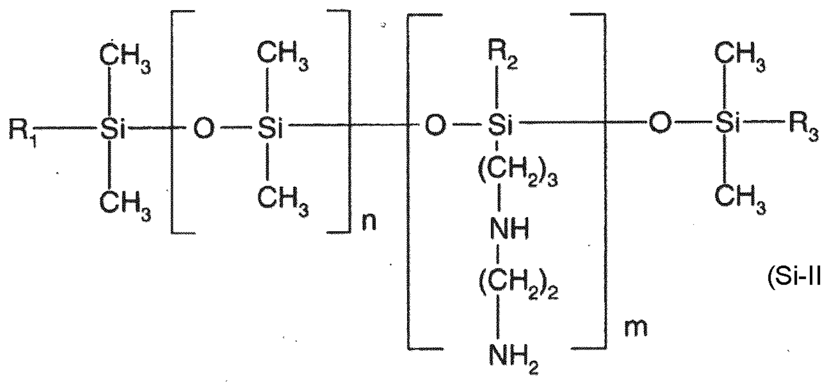

(Si-III)

wobei

- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere erfindungsgemäß bevorzugte Verfahren sind gekennzeichnet durch die Anwendung eines Mittels (a) auf dem keratinischem Material, wobei das Mittel (a) mindestens aminofunktionelles Silikonpolymer (a1) der Formel der Formel (Si-IV) enthält,

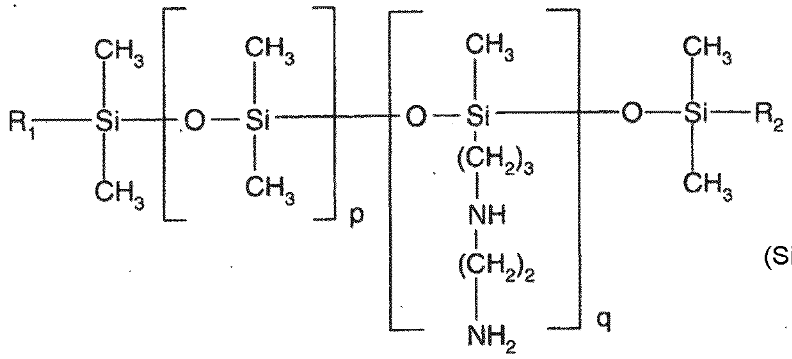

(Si-IV)

in der

- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (Si-III) und (Si-IV) unterscheiden sich durch die Gruppierung am Si-Atom, das die stickstoffhaltige Gruppe trägt: In Formel (Si-III) bedeutet R2 eine Hydroxygruppe oder eine C1-4-Alkoxygruppe, während der Rest in Formel (Si-IV) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (Si-III) und (Si-IV) ist nicht zwingend jedes R1-$Si(CH_3)_2$-Gruppe an eine -[O-$Si(CH_3)_2$]-Gruppierung gebunden.

Als besonders wirkungsvoll im Hinblick auf die gewünschten Effekte haben sich auch erfindungsgemäßen Verfahren erwiesen, in welchen ein Mittel (a) auf den Keratinfasern appliziert wird, welches mindestens ein lineares aminofunktionelles Silikonpolymer (a1) der Formel der Formel (Si-V) enthält

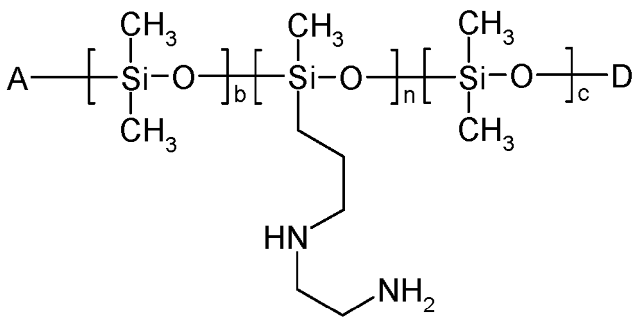

(Si-V),

in der

| | |
|---|---|
| A | für eine Gruppe -OH, $-O-Si(CH_3)_3$,$-O-Si(CH_3)_2OH$ ,$-O-Si(CH_3)_2OCH_3$ steht, |
| D | für eine Gruppe -H, $-Si(CH_3)_3$,$-Si(CH_3)_2OH$, $-Si(CH_3)_2OCH_3$ steht, |
| b, n und c | für ganze Zahlen zwischen 0 und 1000 stehen, |

mit den Maßgaben

- n > 0 und b + c > 0
- mindestens eine der Bedingungen A = -OH bzw. D = -H ist erfüllt.

In der vorstehend genannten Formel (Si-V) sind die einzelnen Siloxaneinheiten mit den Indices b, c und n statistisch verteilt, d.h. es muß sich nicht zwingend um Blockcopolymere handeln.

Das Mittel (a) kann weiterhin auch ein oder mehrere verschiedene aminofunktionalisierte Silikonpolymere enthalten, die durch die Formel (Si-VI)

$$M(R_aQ_bSiO_{(4-a-b)/2)x}(R_cSiO_{(4-c)/2)y}M \qquad \text{(Si-VI)}$$

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel $-R^1HZ$ ist, worin $R^1$ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von $R^1$ schließen Methylen, Ethylen, Propylen, Hexamethylen, Decamethylen,$-CH_2CH(CH_3)CH_2-$, Phenylen, Naphthylen, $-CH_2CH_2SCH_2CH_2-$, $-CH_2CH_2OCH_2-$, $-OCH_2CH_2-$, $-OCH_2CH_2CH_2-$, $-CH_2CH(CH_3)C(O)OCH_2-$, $-(CH_2)_3CC(O)OCH_2CH_2-$, $-C_6H_4C_6H_4-$, $-C_6H_4CH_2C_6H_4-$; und $-(CH_2)_3C(O)SCH_2CH_2-$ ein.

Z ist ein organischer, aminofunktioneller Rest, enthaltend mindestens eine funktionelle Aminogruppe. Eine mögliche Formel für Z ist $NH(CH_2)_zNH_2$, worin z 1 oder mehr ist. Eine andere mögliche Formel für Z ist $-NH(CH_2)_z(CH_2)_{zz}NH$, worin sowohl z als auch zz unabhängig 1 oder mehr sind, wobei diese Struktur Diamino-Ringstrukturen umfaßt, wie Piperazinyl. Z ist am bevorzugtesten ein $-NHCH_2CH_2NH_2$-Rest. Eine andere mögliche Formel für Z ist $-N(CH_2)_z(CH_2)_{zz}NX_2$ oder $-NX_2$, worin jedes X von $X_2$ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel $-CH_2CH_2CH_2NHCH_2CH_2NH_2$. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der $R_aQ_bSiO_{(4-a-b)/2}$-Einheiten zu den $R_cSiO_{(4-c)/2}$-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Im Rahmen einer besonders bevorzugten Ausführungsform ist ein erfindungsgemäßes Verfahren gekennzeichnet durch die Anwendung eines Mittels (a) auf dem keratinischem Material, wobei das Mittel (a) ein aminofunktionelles Silikonpolymer der Formel (Si-VII)

$$R'_aG_{3-a}\text{-}Si(OSiG_2)_n\text{-}(OSiG_bR'_{2-b})_m\text{-}O\text{-}SiG_{3-a}\text{-}R'_a \qquad \text{(Si-VII)},$$

enthält, worin bedeutet:

- G ist-H, eine Phenylgruppe, -OH, $-O-CH_3$, $-CH_3$, $-O-CH_2CH_3$, $-CH_2CH_3$, $-O-CH_2CH_2CH_3$,$-CH_2CH_2CH_3$, $-O-CH(CH_3)_2$, $-CH(CH_3)_2$, $-O-CH_2CH_2CH_2CH_3$,$-CH_2CH_2CH_2CH_3$, $-O-CH_2CH(CH_3)_2$, $-CH_2CH(CH_3)_2$, $-O-CH(CH_3)CH_2CH_3$,$-CH(CH_3)CH_2CH_3$, $-O-C(CH_3)_3$, $-C(CH_3)_3$ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus

  ◦ $-Q-N(R'')-CH_2-CH_2-N(R'')_2$
  ◦ $-Q-N(R'')_2$
  ◦ $-Q-N^+(R'')_3A^-$
  ◦ $-Q-N^+H(R'')_2A^-$
  ◦ $-Q-N^+H_2(R'')A^-$
  ◦ $-Q-N(R'')-CH_2-CH_2-N'R''H_2A^-$,

wobei jedes Q für eine chemische Bindung, $-CH_2-$, $-CH_2-CH_2-$, $-CH_2CH_2CH_2-$, $-C(CH_3)_2-$, $-CH_2CH_2CH_2CH_2-$, $-CH_2C(CH_3)_2-$, $-CH(CH_3)CH_2CH_2-$ steht,
R" für gleiche oder verschiedene Reste aus der Gruppe -H, -Phenyl, -Benzyl, $-CH_2-CH(CH_3)Ph$, der $C_{1-20}$-Alkylreste, vorzugsweise $-CH_3$, $-CH_2CH_3$, $-CH_2CH_2CH_3$,$-CH(CH_3)_2$, $-CH_2CH_2CH_2H_3$, $-CH_2CH(CH_3)_2$, $-CH(CH_3)CH_2CH_3$, $-C(CH_3)_3$, steht und A ein Anion repräsentiert, welches vorzugsweise ausgewählt ist aus Chlorid, Bromid, Iodid oder Methosulfat.

Im Rahmen einer weiteren bevorzugten Ausführungsform ist ein erfindungsgemäßes Verfahren gekennzeichnet durch die Anwendung eines Mittels (a) auf dem keratinischem Material, wobei das Mittel (a) mindestens ein aminofunktionelles Silikonpolymer (a1) der Formel (Si-VIIa) enthält,

$$(CH_3)_3Si-[O-Si(CH_3)_2]_n[OSi(CH_3)]_m-OSi(CH_3)_3 \qquad \text{(Si-VIIa),}$$

$$|$$

$$CH_2CH(CH_3)CH_2NH(CH_2)_2NH_2$$

worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Im Rahmen einer weiteren bevorzugten Ausführungsform ist ein erfindungsgemäßes Verfahren gekennzeichnet durch die Anwendung eines Mittels (a) auf dem keratinischem Material, wobei das Mittel (a) mindestens ein aminofunktionelles Silikonpolymer der Formel (Si-VIIb) enthält

$$R-[Si(CH_3)_2-O]_{n1}[Si(R)-O]_m-[Si(CH_3)_2]_{n2}-R \qquad \text{(Si-VIIb),}$$

$$|$$

$$(CH_2)_3NH(CH_2)_2NH_2$$

enthalten, worin R für -OH, $-O-CH_3$ oder eine $-CH_3$-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese aminofunktionalisierten Siliconpolymere werden nach der INCI-Deklaration als Amodimethicone bezeichnet.

Unabhängig davon, welche aminofunktionellen Silicone eingesetzt werden, sind erfindungsgemäße Mittel (a) bevorzugt, die ein aminofunktionelles Silikonpolymer enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Es hat sich als besonders vorteilhaft herausgestellt, wenn das erfindungsgemäße Mittel das oder die linearen aminofunktionalisierten Silikonpolymere (a1) in bestimmten Mengenbereichen enhält.

Besonders gute Ergebnisse konnten erhalten werden, wenn das Mittel - bezogen auf das Gesamtgewicht des Mittels - ein oder mehrere lineare aminofunktionalisierte Silikonpolymere in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

Im Rahmen einer weiteren besonders bevorzugten Ausführungsform ist ein erfindungsgemäßes Mittel dadurch gekennzeichnet, dass es - bezogen auf das Gesamtgewicht des Mittels - ein oder mehrere lineare aminofunktionalisierte Silikonpolymere (a1) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

Pigmente (a2)

Als zweiten wesentlichen Bestandteil enthält das erfindungsgemäße Mittel mindestens Pigment.

Unter Pigmenten im Sinne der vorliegenden Erfindung werden farbgebende Verbindungen verstanden, welche bei 25 °C in Wasser eine Löslichkeit von weniger als 0,05 g/L besitzen. Die Wasserlöslichkeit kann beispielsweise mittels

der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist ein erfindungsgemäßes Mittel dadurch gekennzeichnet, dass es mindestens eine farbgebende Verbindung (a2) aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Mittel dadurch gekennzeichnet, dass es mindestens eine farbgebende Verbindung (a2) aus der Gruppe der anorganischen Pigmente enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es (a) mindestens eine farbgebende Verbindung (a2) aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona®, Colorona®, Xirona®, Dichrona® und Timiron® von der Firma Merck, Ariabel® und Unipure® von der Firma Sensient, Prestige® von der Firma Eckart Cosmetic Colors und Sunshine® von der Firma Sunstar erhältlich.

Ganz besonders bevorzugte Farbpigmente mit der Handelsbezeichnung Colorona® sind beispielsweise:

Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)

Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona® sind beispielsweise:

Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure® beispielsweise:

Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem Mittel (a) des erfindungsgemäßen Verfahrens ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße $D_{50}$ von 1,0 bis 50 $\mu$m, vorzugsweise von 5,0 bis 45 $\mu$m, bevorzugt von 10 bis 40 $\mu$m, insbesondere von 14 bis 30 $\mu$m, aufweist. Die mittlere Teilchengröße $D_{50}$ kann beispielsweise unter Verwendung von dynamischer Lichtstreuung (DLS) bestimmt werden.

Die (a2) Pigmente stellen den zweiten wesentlichen des erfindungsgemäßen Mittels dar und werden bevorzugt in bestimmten Mengenbereichen im Mittel eingesetzt.

Besonders gute Ergebnisse wurden erhalten, wenn das Mittel - bezogen auf das Gesamtgewicht des Mittels - ein oder mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthielt.

In einer weiteren ganz besonders bevorzugten Ausführungsform ist ein erfindungsgemäßes Mittel dadurch gekennzeichnet, dass das Mittel - bezogen auf das Gesamtgewicht des Mittels - ein oder mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

Als weiteren optionalen Bestandteil können die erfindungsgemäßen Mittel auch einen oder mehrere direktziehende Farbstoffe enthalten. Bei direktziehenden Farbstoffen handelt es sich um Farbstoffe, die direkt auf das Haar

aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L.

Direktziehende Farbstoffe können in anionische, kationische und nichtionische direktziehende Farbstoffe unterteilt werden.

In einer weiteren Ausführungsform ist ein erfindungsgemäßes Verfahren dadurch gekennzeichnet, dass das Mittel (a) mindestens eine farbgebende Verbindung (a2) aus der Gruppe der anionischen, nichtionischen und kationischen direktziehenden Farbstoffe enthält.

Geeignete kationische direktziehende Farbstoffe sind beispielsweise Basic Blue 7, Basic Blue 26, HC Blue 16, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76.

Als nichtionische direktziehende Farbstoffe können beipsielsweise nichtionische Nitro- und Chinonfarbstoffe und neutrale Azofarbstoffe eingesetzt werden. Geeigente nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung ($-SO_3H$) besitzen. In Abhängigkeit vom pH-Wert liegen die protonierten Formen (-COOH, $-SO_3H$) der Carbonsäure- bzw. Sulfonsäuregruppierungen mit ihren deprotonierten Formen ($-COO^-$, $-SO_3^-$ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Im Rahmen einer weiteren Ausführungsform ist ein Verfahren zum Färben von keratinischem Material dadurch gekennzeichnet ist, dass das Mittel (a) mindestens einen anionischen direktziehenden Farbstoff enthält, der ausgewählt ist aus der Gruppe der Nitrophenylendiamine, der Nitroaminophenole, der Azofarbstoffe, der Anthrachinonfarbstoffe, der Triarylmethanfarbstoffe, der Xanthen-Farbstoffe, der Rhodamin-Farbstoffe, der Oxazinfarbstoffen und/oder der Indophenolfarbstoffe, wobei die Farbstoffe aus der vorgenannten Gruppe jeweils mindestens eine Carbonsäuregruppe (-COOH), eine Natriumcarboxylatgruppe (-COONa), eine Kaliumcarboxylatgruppe (-COOK), eine Sulfonsäuregruppe ($-SO_3H$) eine Natriumsulfonatgruppe ($-SO_3Na$) und/oder eine Kaliumsulfonatgruppe ($-SO_3K$) besitzen.

Als geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403, CI 10316, COLIPA n° B001), Acid Yellow 3 (COLIPA n° : C 54, D&C Yellow N° 10, Quinoline Yellow, E104, Food Yellow 13), Acid

Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, Iodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%. Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

In einer weiteren Ausführungsform ist ein erfindungsgemäßes Mittel daher dadurch gekennzeichnet, dass es mindestens einen direktziehenden Farbstoff (a2) enthält, der ausgewählt ist aus der Gruppe aus Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Der oder die direktziehenden Farbstoffe können je nach erwünschter Farbintensität in verschiedenen Mengen

im Mittel eingesetzt werden. Gute Ergebnisse konnten erhalten werden, wenn das Mittel - bezogen auf das Gesamtgewicht des Mittels - ein oder mehrere direktziehende Farbstoffe (a2) in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthält.

Weiterhin kann das Mittel als farbgebende Verbindung (a2) auch mindestens einen photochromen oder thermochromen Farbstoff enthalten.

Photochrome Farbstoffe sind Farbstoffe, die auf Bestrahlung mit UV-Licht (Sonnenlicht oder Schwarzlicht) mit einer reversiblen Farbtonänderung reagieren. Dabei verändert das UV-Licht die chemische Struktur der Farbstoffe und damit ihr Absorptionsverhalten (Photochromie).

Thermochrome Farbstoffe sind Farbstoffe, die auf Temperaturänderungen mit einer reversiblen Farbtonänderung reagieren. Dabei verändert die Temperaturänderung die chemische Struktur der Farbstoffe und damit ihr Absorptionsverhalten (Thermochromie).

Das Mittel kann - bezogen auf das Gesamtgewicht des Mittels - ein oder mehrere photochrome Farbstoffe (a2) in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthalten

Polysaccharide (a3)

Als dritten erfindungswesentlichen Bestandteil (a3) enthalten die erfindungsgemäßen Mittel mindestens ein Polysaccharid, das ausgewählt ist aus der Gruppe aus Hydroxy-$C_1$-$C_{30}$-alkyl-cellulosen, Hydroxy-$C_1$-$C_{30}$-alkylmethylcellulosen, Hydroxy-$C_1$-$C_{30}$-alkylethylcellulosen, $C_1$-$C_{30}$-Alkylcellulosen, $C_6$-$C_{30}$-Alkylhydroxyethylcellulosen, Cellulose, Stärke, Xanthan, Carboxyalkylcellulose, Alginsäure, Carrageen, Tragant, Karaya-Gummi, Gummi Arabicum, Gellan, Pektin, Agaropektin und/oder deren Salzen

Die Bezeichnung Polysaccharide ist die Sammelbezeichnung für makromolekulare Kohlenhydrate, deren Moleküle aus einer großen Zahl glycosidisch miteinander verknüpfter Monosaccharid-Moleküle bestehen (mit einer Molmasse von mind. 5000 g/mol).

Eine besonders gute Eignung zeigten nichtionische Polysaccharide, die ausgewählt sind aus der Gruppe aus Hydroxy-$C_1$-$C_{30}$-alkylcellulosen, Hydroxy-$C_1$-$C_{30}$-alkylmethylcellulosen, Hydroxy-$C_1$-$C_{30}$-alkylethylcellulosen, $C_1$-$C_{30}$-Alkylcellulosen und Cellulose.

Im Rahmen einer besonders bevorzugten Ausführungsform ist ein erfindungsgemäßes Mittel dadurch gekennzeichnet, dass es mindestens ein nichtionisches Polysacharid (a3) enthält, das ausgewählt ist aus der Gruppe aus Hydroxy-$C_1$-$C_{30}$-alkylcellulose, Hydroxy-$C_1$-$C_{30}$-alkylmethyl-cellulose, Hydroxy-$C_1$-$C_{30}$-alkylethylcellulose, $C_1$-$C_{30}$-Alkylcellulose, $C_6$-$C_{30}$-Alkylhydroxyethyl-cellulosen,Cellulose und Stärke.

Hydroxy-$C_1$-$C_{30}$-alkylcellulosen sind Cellulose-Ether, die technisch durch Veretherung von Alkylicellulose mit dem entsprechenden Allkylenoxid hergestellt werden.

Besonders bevorzugte Hydroxy-$C_1$-$C_{30}$-alkylcellulosen sind Hydroxyethylcellulose und Hydroxypropylcellulose.

Hydroxyethylcellulose ist ein Celluloseether, der technisch druch Veretherung von Alkalicellulosem mit Ethylenoxid hergestellt wird.. Da die primäre Hydroxy-Gruppe eines 2-Hydroxyethyl-Substituenten, der beim ersten Veretherungsschritt einer Cellulose-OH-Gruppe mit Ethylenoxid entsteht, mit weiterem Ethylenoxid schneller reagiert als die Hydroxy-Gruppen der Cellulose selbst, liegen in den so entstehenden HEC längere Oligo- oder Polyethylenoxid-Seitenketten neben nicht umgesetzten Cellulose-OH-Gruppen vor.

Eine besonders gut geeignete Hydroxyethylcellulose kann beisielsweise in Form des Rohstoffes Natrosol 250 HHX von der Firma Ashland kommerziell bezogen werden.

Eine weitere besonders gut geeignete Hydroxyethylcellulose wird unter dem Handelsnamen Cellosize WP 300 H von der Firma Dow kommerziell vertrieben.

Hydroxypropylcellulosen sind Celluloseether, die technisch durch Umsetzung von Alkalicellulosen mit Methyloxiran (Propylenoxid) hergestellt werden. Marktübliche Hydroxypropylcellulosen werden mit einem durchschnittlichen molaren Substitutionsgrad (MS) von ca. 4-4,5 angeboten.

Eine besonders gut geeignete Hydroxypropylcellulose kann beisielsweise in Form des Rohstoffes Klucel H CS von der Firma Hercules käuflich erworben werden.

Im Rahmen einer besonders bevorzugten Ausführungsform ist ein erfindungsgemäßes Mittel dadurch gekennzeichnet, dass es mindestens ein Polysacharid (a3) enthält, das ausgewählt ist aus der Gruppe aus Hydroxyethylcellulose und Hydroxypropylcellulose.

Hydroxy-$C_1$-$C_{30}$-alkylmethylcellulosen sind alkoxylierte Methylcellulosen.

Bei einer ganz besonders bevorzugten Hydroxy-$C_1$-$C_{30}$-alkylmethylcellulose handelt es sich beispielsweise um Hydroxyethylmethylcellulose. Hydroxyethylmethylcellulose ist ein Sammelbegriff für chemische Verbindungen, die durch Veretherung von Cellulose hergestellt werden. In diesen ist ein Teil der Hydroxygruppen der Cellulose als Ether mit

Methyl- und mit Hydroxyethyl-Gruppen verknüpft.

Bei einer weiteren ganz besonders bevorzugten Hydroxy-$C_1$-$C_{30}$-alkylmethylcellulose handelt es sich beispielsweise um Hydroxypropylmethylcellulose. Hydroxypropylmethylcellulose ist eine mit Propylenoxid substituierte Methylcellulose. Sie kommt in verschiedenen Polymerisationsgraden und unterschiedlichen Substitutionsgraden auf den Markt.

Eine besonders gut geeignete Hydroxypropyl methyl cellulose kann zum Beispiel in Form des Produktes Benecel K 4 M von der Firma Ashland kommeriell erworben werden.

Hydroxy-$C_1$-$C_{30}$-alkylethylcellulosen sind alkoxylierte Ethylcellulosen.

Beispielhaft als geeignete $C_1$-$C_{30}$-Alkylcellulosen können Methylcellulose und Ethylcellulose genannt werden.

Unter den Handelsnamen Culminal DM 40 und Culminal MC 12000 sind verschiedene Methylcellulosen von der Firme Herkules käuflich erhältlich.

Ethylcellulose wird halb synthetisch aus der natürlich vorkommenden Cellulose gewonnen und ist ein Celluloseether, der in verschiedenen Typen vorkommt, die sich in ihrem Polymerisationsgrad (Molmassenverteilung) und in ihrem Veretherungsgrad unterscheiden.

Eine besonders gut geeignete Ethylcellulose kann beipsielsweise unter dem Handelsnamen Aqualon EC N 10 Ethylcellulose von der Firme Ashland kommerziell bezogen werden.

Unter $C_{6-30}$-Alkylhydroxyethylcellulosen sind solche Celluloseverbindungen zu verstehen, bei welchen hydrophobe $C_{6-30}$-Alkylgruppen über Ethylenoxideinheiten an das Cellulosegerüst gebunden sind. Aufgrund der Einführung von $C_{6-30}$-Alkylgruppen an Ethylenoxideinheiten des Grundgerüsts weisen diese Cellulosen einen hydrophoben Charakter auf und werden auch als hydrophob modifizierte Hydroxyethylcellulosen bezeichnet.

Cellulose ist aus β-1,4-glycosidisch-verknüpften D-Glucopyranose-Einheiten aufgebaut. In festem Zustand alternieren in Cellulose kristalline Regionen mit solchen von geringer Ordnung (amorphe Regionen). Natürliche und herstellungsbedingte Verunreinigungen, wie insbesondere das Vorliegen von Carboxygruppen, liegen typischerweise in Bereich von ca. 1%. Erfindungsgemäß wird Cellulose selbst daher nicht als anionisches Polysaccharid angesehen.

Erfindungsgemäß einsetzbare Cellulose weist einen Polymerisationsgrad (DP), also eine Kettelänge aus Glucopyranose-Einheiten, von 10 bis ca. 8000 auf. Es hat sich jedoch herausgestellt, dass insbesondere Cellulosen mit niedrigen Polymerisationsgrad einen positiven Effekt auf die Färbeeigenschaften der Mittel ausüben.

Insbesondere sogenannte microkristalline Cellulose zeigt hierbei vorteilhafte Effekte. Microkristalline Cellulose wird durch partielle alkalische oder saure Hydrolyse von Cellulosen gewonnen, bei denen nur die amorphen Bereiche der teilkristallinen Cellulose angegriffen und vollständig aufgelöst werden. Es resultiert zunächst microfeine Cellulose, die in wässriger Suspension unter mechanischer Krafteinwirkung in microkristalline Cellulose desaggregiert wird.

Der nach Hydrolyse verbleibende Polymerisationsgrad (auch Levelling-off-Polymerisationsgrad = LODP) der microkristallinen Cellulose liegt im Bereich von ca. 30-400.

Bevorzugte Cellulosen sind daher microkristalline Cellulosen und weisen einen Polymerisationsgrad von 30 bis 400 auf.

Eine Ausführungsform der vorliegenden Erfindung ist daher dadurch gekennzeichnet, dass das Mittel als Cellulose microkristalline Cellulose enthält.

Stärke ist ein Polysaccharid mit der Formel $(C_6H_{10}O_5)_n$, das aus α-D-Glucose-Einheiten besteht. Das Makromolekül zählt zu den Kohlenhydraten. Stärke ist einer der wichtigsten Reservestoffe in pflanzlichen Zellen.

Eine Erfindungsgemäß verwendbare Stärke kann beispielsweise aus Kartoffeln, Mais, Reis, Erbsen, Eicheln, Kastanien, Gerste, Weizen, Bananen, Sago, Hirse, Sorghum, Hafer, Roggen, Bohnen, Batate, Maranta oder Maniok gewonnen werden.

Im Rahmen einer weiteren bevorzugten Ausführungsform ist ein erfindungsgemäßes Mittel dadurch gekennzeichnet, dass es mindestens ein Polysacharid (a3) enthält, das aus der Gruppe ausKartoffelsstärke, Maisstärke, Reisstärke, Erbsenstärke, Eichelstärke, Kastanienstärke, Gerstenstärke, Weizenstärke, Roggenstärke, Bananenstärke, Sagostärke, Hirsestärke, Sorghumstärke, Haferstärke, Bohnenstärke, Batatestärke, Marantastärke und Maniokstärke ausgewählt ist.

Im Rahmen einer besonders bevorzugten Ausführungsform ist ein erfindungsgemäßes Mittel dadurch gekennzeichnet, dass es mindestens ein anionisches Polysaccharid (a3) enthält, dass ausgewählt ist aus der Gruppe aus Xanthan, Carboxyalkylcellulose, Alginsäure, Carrageen, Tragant, Karaya-Gummi, Gummi Arabicum, Gellan, Pektin, Agaropektin und/oder deren Salzen.

Xanthan ist ein Polysaccharid, welches unter anderem aus den Strukturbestandteilen D-Glucose, D-Mannose, D-Glucuronsäure, Acetat und Pyruvat aufgebaut ist und das auch unter dem INCI Namen Xanthan Gum bekannt ist. Xanthan trägt Carboxygruppen und ist anionisch bzw. anionisierbar. Auch die phyiologisch verträglichen Salze von Xanthan sind erfindungsgemäß.

Als Alginate (INCI-Name Algin) werden die Salze der Alginsäure bezeichnet. Alginate sind saure, Carboxy-Gruppen enthaltende Polysaccharide, bestehend aus D-Mannuronsäure und D-Guluronsäure in unterschiedlichen Verhältnissen, welche mit 1-4-glycosidischen Bindungen verknüpft sind. Erfindungsgemäß sind insbesondere die Alkali- als auch die Erdalkalisalze der Alignsäuren. Besonders vorteilhaft hat sich der Einsatz von Alginsäure, Natriumalginat,

Kaliumalginat, Ammoniumalginat und/oder Calciumalginat in den erfindungsgemäßen Zubereitungen herausgestellt.

Carboxyalkylcellulosen sind Celluloseether, bei denen die Wasserstoffatome der Hydroxygruppen der Cellulose teilweise oder vollständig durch Carboxyalkylgruppen substituiert sind. Eine bevorzugte Carboxyalkylcellulose ist die Carboxymethylcellulose, die vorzugsweise in Form ihres Natriumsalzes (Natrium-Carboxymethylcellulose) als anionisches Polymer Einsatz finden kann.

Carrageen wird synonym auch als Carrageenan bezeichnet. Bei Carrageen handelt es sich um sulfatiertes Galactan, das beispielsweise durch Extraktion aus Rotalgen gewonnen werden kann. Das aus dem Heißwasserextrakt der Algen ausgefällte Carrageenan ist ein farbloses bis sandfarbenes Pulver mit einem Molekulargewicht von 100000-800000 und einem Sulfat-Gehalt von ca. 25 %, das in warmem Wasser sehr leicht löslich ist. Beim Carrageenan unterscheidet man drei Hauptbestandteile mit unterschiedlichen Eigenschaften, bei denen es sich um $\kappa$ -Carrageenan, ι-Carrageenan und $\lambda$-Carrageenan handelt. Die gelbildende $\kappa$-Fraktion besteht aus D-Galactose-4-sulfat und 3,6-Anhydro-$\alpha$-D-galactose, die abwechselnd in 1,3- und 1,4-Stellung glycosidisch verbunden sind. Die nicht gelierende $\lambda$-Fraktion ist aus 1,3-glycosidisch verknüpften D-Galactose-2-sulfat- und 1,4-verbundenen D-Galactose-2,6-disulfat-Resten zusammengesetzt und in kaltem Wasser leicht löslich. Das aus D-Galactose-4-sulfat in 1,3-Bindung und 3,6-Anhydro-$\alpha$-D-galactose-2-sulfat in 1,4-Bindung aufgebaute i-Carrageenan ist in heißem Wasser löslich und auch gelbildend.

Innerhalb der Gruppe der anionischen Polysaccharide hat sich Xanthan als ganz besonders gut geeignete zur Erzielung von intensiven Farbergebnissen erwiesen. Erfindungsgemäße Mittel enthaltend Xanthan (a3) sind daher explizit ganz besonders bevorzugt.

Im Rahmen einer explizit ganz besonders bevorzugten Ausführungsform ist ein erfindungsgemäßes Mittel dadurch gekennzeichnet, dass es mindestens ein Xanthan (a3) enthält.

Das oder die Polysaccharide, inbesondere die zuvor beschriebenen bevorzugten und besonders bevorzugten Vertreter, werden zur Erzielung einer bestmöglichen Farbintensität bevorzugt in bestimmten Mengenbereichen im erfindungsgemäßen Mittel eingesetzt.

In diesem Zusammenhang hat sich als ganz besonders bevorzugt herausgestellt, wenn das erfindungsgemäße Mittel - bezogen auf das Gesamtgewicht des Mittels - ein oder mehrere Polysaccharide (a3) in einer Gesamtmenge von 0,1 bis 6,0 Gew.-%, bevorzugt 0,5 bis 5,0 Gew.-%, weiter bevorzugt von 1,0 bis 4,0 Gew.-% und ganz besonders bevorzugt von 1,5 bis 3,5 Gew.-% enthält.

Im Rahmen einer besonders bevorzugten Ausführungsform ist ein erfindungsgemäßes Mittel dadurch gekennzeichnet, dass es - bezogen auf das Gesamtgewicht des Mittels - ein oder mehrere Polysaccharide (a3) in einer Gesamtmenge von 0,1 bis 6,0 Gew.-%, bevorzugt 0,5 bis 5,0 Gew.-%, weiter bevorzugt von 1,0 bis 4,0 Gew.-% und ganz besonders bevorzugt von 1,5 bis 3,5 Gew.-% enthält.

Im Rahmen einer besonders bevorzugten Ausführungsform ist ein erfindungsgemäßes Mittel dadurch gekennzeichnet, dass es - bezogen auf das Gesamtgewicht des Mittels - ein oder mehrere nichtionischePolysaccharide (a3) in einer Gesamtmenge von 0,1 bis 6,0 Gew.-%, bevorzugt 0,5 bis 5,0 Gew.-%, weiter bevorzugt von 1,0 bis 4,0 Gew.-% und ganz besonders bevorzugt von 1,5 bis 3,5 Gew.-% enthält.

Im Rahmen einer besonders bevorzugten Ausführungsform ist ein erfindungsgemäßes Mittel dadurch gekennzeichnet, dass es - bezogen auf das Gesamtgewicht des Mittels - ein oder mehrere anionische Polysaccharide (a3) in einer Gesamtmenge von 0,1 bis 6,0 Gew.-%, bevorzugt 0,5 bis 5,0 Gew.-%, weiter bevorzugt von 1,0 bis 4,0 Gew.-% und ganz besonders bevorzugt von 1,5 bis 3,5 Gew.-% enthält.

Es ist explizit ganz besonders bevorzugt, wenn das erfindungsgemäße Mittel - bezogen auf das Gesamtgewicht des Mittels - e 0,1 bis 6,0 Gew.-%, bevorzugt 0,5 bis 5,0 Gew.-%, weiter bevorzugt von 1,0 bis 4,0 Gew.-% und ganz besonders bevorzugt von 1,5 bis 3,5 Gew.-% Hydroxyethylcellulose enthält.

Es ist weiterhin explizit ganz besonders bevorzugt, wenn das erfindungsgemäße Mittel - bezogen auf das Gesamtgewicht des Mittels - e 0,1 bis 6,0 Gew.-%, bevorzugt 0,5 bis 5,0 Gew.-%, weiter bevorzugt von 1,0 bis 4,0 Gew.-% und ganz besonders bevorzugt von 1,5 bis 3,5 Gew.-% Hydroxypropylcellulose enthält.

Es ist weiterhin explizit ganz besonders bevorzugt, wenn das erfindungsgemäße Mittel - bezogen auf das Gesamtgewicht des Mittels - e 0,1 bis 6,0 Gew.-%, bevorzugt 0,5 bis 5,0 Gew.-%, weiter bevorzugt von 1,0 bis 4,0 Gew.-% und ganz besonders bevorzugt von 1,5 bis 3,5 Gew.-% Xanthan enthält.

Verzicht auf Silikonharze im Mittel

Die zu dieser Erfindung führenden Arbeiten haben gezeigt, dass die zuvor beschriebenen Mittel, die zusätzlich mindestens ein Silkonharz enthalten, im Hinblick auf die Erzeugung von Färbungen mit hoher Farbintensität signifikante Nachteile besitzen. Aus diesem Grund ist das erfindungsgemäße Mittel dadurch gekennzeichnet, dass es frei ist von Silkonharzen.

Unter einem Silikonharz im Sinne der erifndung wird ein MX Harz verstanden, wobei "M" für eine Einheit $Me_3SiO$ steht und "Q" für eine Einheit $SiO_4$ steht. Silkonharze umfassen damit mindestens eine, bevorzugte mehrere, "M"-Einheiten und mindestens eine, bevorzugt mehrere Q-Einheiten. Bedingt durch die SiO4-Einheit sind Silikonharze somit im Gegensatz zu den linearen Silikonpolymeren (a1) verzweigt.

Betreffend die weitere Definition der Silkonharze wird vollinhaltlich auf die Schrift EP 3058935 B1 verwiesen.

Anders ausgedrückt ist ein der Gegenstand der vorliegenden Erfindung ist ein Mittel zum Färben von keratinischem Material, insbesondere menschlichen Haaren, enthaltend

(a1) mindestens ein lineares, aminofunktionalisiertes Silikonpolymer, und
(a2) mindestens ein Pigment mit einer Löslichkeit in Wasser bei 25 °C von weniger als 0,05 g/L, und
(a3) mindestens ein Polysaccharid, das ausgewählt ist aus der Gruppe aus Hydroxy-$C_1$-$C_{30}$-alkylcellulosen, Hydroxy-$C_1$-$C_{30}$-alkylmethylcellulosen, Hydroxy-$C_1$-$C_{30}$-alkylethylcellulosen, $C_1$-$C_{30}$-Alkylcellulosen, $C_6$-$C_{30}$-Alkylhydroxyethylcellulosen, Cellulose, Stärke, Xanthan, Carboxyalkylcellulose, Alginsäure, Carrageen, Tragant, Karaya-Gummi, Gummi Arabicum, Gellan, Pektin, Agaropektin und/oder deren Salzen,
dadurch gekennzeichnet, dass das Mittel frei ist von MQ-Silikonharzen

Beispiele für Silikonharze sind beipsielsweise von Wacker-Chemie AG, D-81737 München kommerziell erhältlich.

Zum Beispiel ist das MQ-Silikonharz POWDER 803 TF das Produkt einer Co-Hydrolyse von Tetraalkoxy-silan (Q-Einheit) und Trimethyl-ethoxysilan (M-Einheit) und kann als dreidimensionales Netzwerk einer Polykieselsäure verstanden werden, die an den Enden mit Trimethylsilyl-Gruppierungen beblockt ist.

Weitere MQ-Silikonharze können auch von Dow Corning bezogen werden. Zum Beispiel ist Dow Corning® MQ-1640 Flake Resin eine Kombination von MQ und T Propyl-Silikon-Harz und besitzt den INCI Namen: Trimethylsiloxysilicate (and) Polypropyl silsesquioxane.

Eine Mischung aus Aminosilikon und MQ-Harz kann beipsielsweise auch in Form des Handelsproduktes Belsil ADM 8301 E von Wacker kommerziell erworben werden, diese Substanz trägt den INCI-Namen Amodimethicone/Morpholinomethyl Silsesquioxane Copolymer.

Kennzeichnend für die erfindungsgemäßen Mittel dass es die zuvor beschriebenen MQ-Harze nicht enthält, d.h. die Gesamtmenge aller im erifndungsgemäßen Mittel enthaltenen MQ-Silikonharze liegt - bezogen auf die Gesamtmenge des Mittels - bei 0 Gew.-%.

Fettbestandteile (a4)

Als weiteren optionalen Bestandteil kann das erfindungsgemäße Mittel zusätzlich mindestens einen Fettbestandteil (a4) enthalten. Es hat sich herausgestellt, dass der Einsatz mindestens eines Fettbestandteils (a4) dazu führt, dass das Mittel in Form einer Emulsion vorliegt, welche die optimale Viskosität besitzt und sich auch im Hinblick auf die Verbesserung der Farbintensität als vorteilhaft herausgestellt hat.

Unter "Fettbestandteilen" werden im Sinne der Erfindung organische Verbindungen mit einer Löslichkeit in Wasser bei Raumtemperatur (22 °C) und atmosphärischem Druck (760 mmHg) von weniger als 1 Gew.-%, bevorzugt von weniger als 0,1 Gew.-% verstanden. Unter die Definition der Fettbestandteile fallen explizit nur ungeladene (d.h. nichtionische) Verbindungen. Fettbestandteile besitzen mindestens eine gesättigte oder ungesättigte Alkylgruppe mit mindestens 12 C-Atomen. Das Molgewicht der Fettbestandteile liegt bei maximal 5000 g/mol, bevorzugt bei maximal 2500 g/mol und besonders bevorzugt bei maximal 1000 g/mol. Bei den Fettbestandteilen handelt es sich weder um polyoxyalkylierte noch um polyglycerylierte Verbindungen.

Ganz besonders bevorzugt werden die im Mittelenthaltenen Fettbestandteile (a4) ausgewählt aus der Gruppe der $C_{12}$-$C_{30}$-Fettalkohole, der $C_{12}$-$C_{30}$-Fettsäuretriglyceride, der $C_{12}$-$C_{30}$-Fettsäure-monoglyceride, der $C_{12}$-$C_{30}$-Fettsäurediglyceride und/oder der Kohlenwasserstoffe.

Im Rahmen einer weiteren bevorzugten Ausführungsform ist ein erfindungsgemäßes Mittel dadurch gekennzeichnet, dass es einen oder mehrere Fettbestandteile (a4) aus der Gruppe der $C_{12}$-$C_{30}$-Fettalkohole, der $C_{12}$-$C_{30}$-Fettsäuretriglyceride, der $C_{12}$-$C_{30}$-Fettsäuremonoglyceride, der $C_{12}$-$C_{30}$-Fettsäurediglyceride und/oder der Kohlenwasserstoffe enthält.

Als ganz besonders bevorzugte Fettbestandteile werden in diesem Zusammenhang die Bestandteile aus der Gruppe der $C_{12}$-$C_{30}$-Fettalkohole, der $C_{12}$-$C_{30}$-Fettsäuretriglyceride, der $C_{12}$-$C_{30}$-Fettsäuremonoglyceride, der $C_{12}$-$C_{30}$-Fettsäurediglyceride und/oder der Kohlenwasserstoffe verstanden. Im Sinne der vorliegenden Erfindung werden explizit nur nichtionische Substanzen als Fettbestandteile betrachtet. Geladene Verbindungen wie beispielsweise Fettsäuren und ihre Salze werden nicht als Fettbestandteil verstanden.

Bei den $C_{12}$-$C_{30}$-Fettalkoholen kann es sich um gesättigte, ein- oder mehrfach ungesättigte, lineare oder verzweigte Fettalkohole mit 12 bis 30 C-Atomen handeln.

Beispiele für bevorzugte lineare, gesättigte $C_{12}$-$C_{30}$-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Bevorzugte lineare, ungesättigte Fettalkohole sind (9*Z*)-Octadec-9-en-1-ol (Oleylalkohol), (9E)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenoylalkohol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Erucylalkohol ((132)-Docos-13-en-1-ol) und/oder Brassidylalkohol ((13E)-Docosen-1-ol).

Die bevorzugten Vertreter für verzweigte Fettalkohole sind 2-Octyl-dodecanol, 2-Hexyl-Dodecanol und/oder 2-Butyl-dodecanol.

Im Rahmen einer weiteren bevorzugten Ausführungsform ist ein erfindungsgemäßes Mittel dadurch gekennzeichnet, dass es einen oder mehrere $C_{12}$-$C_{30}$-Fettalkohole (a4) aus der Gruppe aus

Dodecan-1-ol (Dodecylalkohol, Laurylalkohol),
Tetradecan-1-ol (Tetradecylalkohol, Myristylalkohol),
Hexadecan-1-ol (Hexadecylalkohol, Cetylalkohol, Palmitylalkohol),
Octadecan-1-ol (Octadecylalkohol, Stearylalkohol),
Arachylalkohol (Eicosan-1-ol),
Heneicosylalkohol (Heneicosan-1-ol),
Behenylalkohol (Docosan-1-ol),
(9*Z*)-Octadec-9-en-1-ol (Oleylalkohol),
(9*E*)-Octadec-9-en-1-ol (Elaidylalkohol),
(9*Z*,12*Z*)-Octadeca-9,12-dien-1-ol (Linoleylalkohol),
(9*Z*,12*Z*,15*Z*)-Octadeca-9,12,15-trien-1-ol (Linolenoylalkohol),
Gadoleylalkohol ((9*Z*)-Eicos-9-en-1-ol),
Arachidonalkohol ((5*Z*,8*Z*,11*Z*,14*Z*)-Eicosa-5,8,11,14-tetraen-1-ol),
Erucylalkohol ((13*Z*)-Docos-13-en-1-ol),
Brassidylalkohol ((13E)-Docosen-1-ol),
2-Octyl-dodecanol,
2-Hexyl-Dodecanol und/oder
2-Butyl-dodecanol enthält.

Es hat sich als ganz besonders bevorzugt erwiesen, ein oder mehrere $C_{12}$-$C_{30}$-Fettalkohole (a4) in ganz bestimmten Mengenbereichen einzusetzen.

Es ist ganz besonders bevorzugt, wenn die das Mittel - bezogen auf das Gesamtgewicht des Mittels einen oder mehrere $C_{12}$-$C_{30}$-Fettalkohole (a4) in einer Gesamtmenge von 2,0 bis 50,0 Gew.-%, bevorzugt von 3,0 bis 30,0 Gew.-%, weiter bevorzugt von 4,0 bis 20,0 Gew.-%, noch weiter bevorzugt von 5,0 bis 15,0 Gew.-% und ganz besonders bevorzugt von 5,0 bis 10,0 Gew.-% enthält.

Weiterhin als ganz geeigneten Fettbestandteil (a4) kann das Mittel auch mindestens ein $C_{12}$-$C_{30}$-Fettsäuretriglycerid, der $C_{12}$-$C_{30}$-Fettsäuremonoglycerid und/oder $C_{12}$-$C_{30}$-Fettsäurediglycerid enthalten. Unter einem $C_{12}$-$C_{30}$-Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte $C_{12}$-$C_{30}$-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Es zeichnen sich die Fettsäuretriglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)- Tetracos-15-ensäure].

Die Fettsäuretriglyceride können auch natürlichen Ursprungs sein. Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Fettsäure-Triglyceride bzw. deren Gemische sind zum Einsatz im erfindungsgemäßen Produkt besonders geeignet.

Unter einem $C_{12}$-$C_{30}$-Fettsäuremonoglycerid wird der Monoester des dreiwertigen Alkohols Glycerin mit einem

Äquivalent Fettsäure verstanden. Hierbei kann entweder die mittlere Hydroxygruppe des Glycerins oder die endständige Hydroxygruppe des Glycerins mit der Fettsäure verestert sein.

Es zeichnen sich die $C_{12}$-$C_{30}$-Fettsäuremonoglycerid durch besondere Eignung aus, bei welchen eine Hydroxygruppe des Glycerins mit einer Fettsäure verestert wird, wobei die Fettsäuren ausgewählt sind aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] oder Nervonsäure [(15Z)- Tetracos-15-ensäure].

Unter einem $C_{12}$-$C_{30}$-Fettsäurediglycerid wird der Diester des dreiwertigen Alkohols Glycerin mit zwei Äquivalenten Fettsäure verstanden. Hierbei können entweder die mittlere und eine endständige Hydroxygruppe des Glycerins mit zwei Äquivalenten Fettsäure verestert sein, oder aber beide endständigen Hydroxygruppen des Glycerins sind mit jeweils einer Fettsäure verestert. Das Glycerin kann hierbei sowohl mit zwei strukturgleichen als auch mit zwei unterschiedlichen Fettsäuren verestert sein.

Es zeichnen sich die Fettsäurediglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Ganz besonders gute Ergebnisse wurden erhalten, wenn die Zusammensetzung (B) mindestens ein $C_{12}$-$C_{30}$-Fettsäuremonoglycerid enthielt, welches ausgewählt ist aus den Monoestern von Glycerin mit einem Äquivalent Fettsäure aus der Gruppe aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)- Tetracos-15-ensäure].

Im Rahmen einer weiteren Ausführungsform ist ein erfindungsgemäßes Mittel dadurch gekennzeichnet, dass es mindestens ein $C_{12}$-$C_{30}$-Fettsäuremonoglycerid (a4) enthält, welches ausgewählt ist aus den Monoestern von Glycerin mit einem Äquivalent Fettsäure aus der Gruppe aus Dodecansäure, Tetradecansäure, Hexadecansäure, Tetracosansäure, Octadecansäure, Eicosansäure und/oder Docosansäure.

Es hat sich als bevorzugt erwiesen, ein oder mehrere $C_{12}$-$C_{30}$-Fettsäuremono-, $C_{12}$-$C_{30}$-Fettsäuredi- und/oder $C_{12}$-$C_{30}$-Fettsäuretriglyceride (a4) in ganz bestimmten Mengenbereichen im Mittel einzusetzen.

Im Hinblick auf die Lösung der erfindungsgemäßen Aufgabenstellung hat es sich als vorteilhaft erwiesen, wenn das Mittel - bezogen auf das Gesamtgewicht des Mittels - ein oder mehrere $C_{12}$-$C_{30}$-Fettsäuremono-, $C_{12}$-$C_{30}$-Fettsäuredi- und/oder $C_{12}$-$C_{30}$-Fettsäuretriglyceride (a4) in einer Gesamtmenge von 0,1 bis 20,0 Gew.-%, bevorzugt 0,3 bis 15,0 Gew.-%, weiter bevorzugt 0,5 bis 10,0 Gew.-% und ganz besonders bevorzugt von 0,8 bis 5,0 Gew.-% enthielt.

Im Rahmen einer ganz besonders bevorzugten Ausführungsform ist ein erfindungsgemäßes Verfahren dadurch gekennzeichnet, dass das Mittel- bezogen auf das Gesamtgewicht des Mittelsein oder mehrere $C_{12}$-$C_{30}$-Fettsäuremono-, $C_{12}$-$C_{30}$-Fettsäuredi- und/oder $C_{12}$-$C_{30}$-Fettsäure-triglyceride in einer Gesamtmenge von 0,1 bis 20,0 Gew.-%, bevorzugt 0,3 bis 15,0 Gew.-%, weiter bevorzugt 0,5 bis 10,0 Gew.-% und ganz besonders bevorzugt von 0,8 bis 5,0 Gew.-% enthält.

Die $C_{12}$-$C_{30}$-Fettsäuremono-, $C_{12}$-$C_{30}$-Fettsäuredi- und/oder $C_{12}$-$C_{30}$-Fettsäuretriglyceride können als alleinige Fettbestandteile (a4) in den Mitteln eingesetzt werden. Es kann sich jedoch auch erfindungsgemäß, mindestens ein $C_{12}$-$C_{30}$-Fettsäuremono-, $C_{12}$-$C_{30}$-Fettsäuredi- und/oder $C_{12}$-$C_{30}$-Fettsäuretriglycerid in Kombination mit mindestens einem $C_{12}$-$C_{30}$-Fettalkohol in das Mittel einzuarbeiten.

Weiterhin als ganz besonders bevorzugten Fettbestandteil (a4) kann das Mittel auch mindestens einen Kohlenwasserstoff enthalten.

Kohlenwasserstoffe sind ausschließlich aus den Atomen Kohlenstoff und Wasserstoff bestehende Verbindungen mit 8 bis 80 C-Atomen. Bevorzugt sind in diesem Zusammenhang insbesondere aliphatische Kohlenwasserstoffe wie beispielsweise Mineralöle, flüssige Paraffinöle (z.B. Paraffinium Liquidum oder Paraffinum Perliquidum), Isoparaffinöle, halbfeste Paraffinöle, Paraffinwachse, Hartparaffin (Paraffinum Solidum), Vaseline und Polydecene.

Als besonders geeignet haben sich in diesem Zusammenhang flüssige Paraffinöle (Paraffinum Liquidum und Paraffinium Perliquidum) erwiesen. Ganz besonders bevorzugt handelt es sich bei dem Kohlenwasserstoff um Paraffinum

Liquidum, auch Weißöl genannt. Bei Paraffinum Liquidum handelt es sich um ein Gemisch gereinigter, gesättigter, aliphatischer Kohlenwasserstoffe, das größtenteils aus Kohlenwasserstoffketten mit einer C-Kettenverteilung von 25 bis 35 C-Atomen besteht.

Ganz besonders gute Ergebnisse wurden erhalten, wenn das Mittel mindestens einen Kohlenwasserstoff (a4) enthielt, der ausgewählt ist aus der Gruppe der Mineralöle, der flüssige Paraffinöle, Isoparaffinöle, halbfeste Paraffinöle, Paraffinwachse, Hartparaffin (Paraffinum Solidum), Vaseline und Polydecene.

Im Rahmen einer ganz besonders bevorzugten Ausführungsform ist ein erfindungsgemäßes Mittel dadurch gekennzeichnet, dass es mindestens einen Fettbestandteil (a4) aus der Gruppe der Kohlenwasserstoffe enthält.

Im Hinblick auf die Lösung der erfindungsgemäßen Aufgabenstellung hat es sich als ganz besonders bevozugt erwiesen, wenn das Mittel - bezogen auf das Gesamtgewicht des Mittels - einen oder mehrere Kohlenwasserstoffe (a4) in einer Gesamtmenge von 0,5 bis 20,0 Gew.-%, bevorzugt 0,7 bis 10,0 Gew.-%, weiter bevorzugt von 0,9 bis 5,0 Gew.-% und ganz besonders bevorzugt von 1,0 bis 4,0 Gew.-% enthielt.

Im Rahmen einer ganz besonders bevorzugten Ausführungsform ist ein erfindungsgemäßes Mittel dadurch gekennzeichnet, dass es - bezogen auf das Gesamtgewicht des Mittels - einen oder mehrere Kohlenwasserstoffe (a4) in einer Gesamtmenge von in einer Gesamtmenge von 0,5 bis 20,0 Gew.-%, bevorzugt 0,7 bis 10,0 Gew.-%, weiter bevorzugt von 0,9 bis 5,0 Gew.-% und ganz besonders bevorzugt von 1,0 bis 4,0 Gew.-% enthält.

Der oder die Kohlenwasserstoffe können als alleinige Fettbestandteile (a4) in den Mitteln eingesetzt werden. Es ist jedoch ebenfalls erfindungsgemäß, mindestens einen Kohlenwasserstoff in Kombination mit mindestens einem weiteren Bestandteil in die Mittel einzuarbeiten.

Ganz besonders bevorzugt enthält das Mittel mindestens einen Fettbestandteil (a4) aus der Gruppe der $C_{12}$-$C_{30}$-Fettalkohole und mindestens einen weiteren Fettbestandteil aus der Gruppe der Kohlenwasserstoffe.

Wassergehalt im Mittel

Bei dem zuvor beschriebenen Mittel handelt es sich um ein anwendungsbereites Mittel, welches auf das keratinische Material appliziert werden kann. Dieses anwendungsbereite Mittel besitzt bevorzugt einen hohen Wasseranteil. Es hat sich herausgestellt, dass besonders die Mittel besonders gut geeignet sind, die - bezogen auf das Gesamtgewicht des Mittels - 50,0 bis 98,0 Gew.-%, bevorzugt 60,0 bis 90,0 Gew.-%, weiter bevorzugt 70,0 bis 90,0 Gew.-% und ganz besonders bevorzugt 75,0 bis 90,0 Gew.-% Wasser enthalten.

In einer weiteren explizit ganz besonders bevorzugten Ausführungsform ist ein erfindungsgemäßes Mittel dadurch gekennzeichnet, dass es - bezogen auf das Gesamtgewicht des Mittels - 50,0 bis 98,0 Gew.-%, bevorzugt 60,0 bis 90,0 Gew.-%, weiter bevorzugt 70,0 bis 90,0 Gew.-% und ganz besonders bevorzugt 75,0 bis 90,0 Gew.-% Wasser enthält.

Tenside im Mittel

Bedingt durch ihren Gehalt an Wasser und Fettbestandteil (a4) liegt das erfindungsgemäße Mittel besonders bevorzugt in Form einer Emulsion vor. Um die Ausbildung der Emulsion weiter zu optimieren, hat es sich als ganz besonders bevorzugt erwiesen, im Mittel weiterhin mindestens ein Tensid einzusetzen.

Ganz besonders bevorzugt enthält das Mittel deshalb zusätzlich mindestens ein Tensid.

Im Rahmen einer weiteren besonders bevorzugten Ausführungsform ist ein erfindungsgemäßes Mittel dadurch gekennzeichnet, dass es mindestens ein Tensid, enthält.

Unter dem Begriff Tenside (T) werden grenzflächenaktive Substanzen, die an Ober- und Grenzflächen Adsorptionsschichten bilden oder in Volumenphasen zu Mizell-Kolloiden oder lyotropen Mesophasen aggregieren können, verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Im Rahmen einer ganz besonders bevorzugten Ausführungsform ist ein erfindungsgemäßes Mittel dadurch gekennzeichnet, dass es mindestens ein anionisches und/oder nichitonisches Tensid enthält.

Zu den geeigneten anionischen Tensiden, die in den erfindungsgemäßen Mitteln eingesetzt werden können, zählen:

- lineare und verzweigte Fettsäuren mit 8 bis 30 C-Atomen (Seifen),
- Ethercarbonsäuren der Formel $R-O-(CH_2-CH_2O)_x-CH_2-COOH$, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,

- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel $R\text{-}(OCH_2\text{-}CH_2)_x\text{-}OSO_3^-\ X^+$, in der R eine bevorzugt lineare oder verzweigte, gesättigte oder ungesättigte Alkylgruppe mit 8 bis 30 C-Atomen, x = 0 oder 1 bis 12 und X ein Alkali- oder Ammoniumion ist,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel,

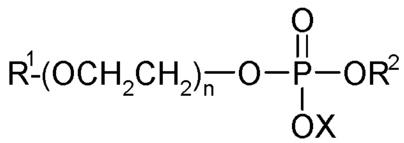

in der $R^1$ bevorzugt für einen aliphatischen Kohlenwasserstoffrest mit 8 bis 30 Kohlenstoffatomen, $R^2$ für Wasserstoff, einen Rest $(CH_2CH_2O)_nR^1$ oder X, n für Zahlen von 0 bis 10 und X für Wasserstoff, ein Alkali- oder Erdalkalimetall oder $NR^3R^4R^5R^6$, mit $R^3$ bis $R^6$ unabhängig voneinander stehend für einen $C_1$ bis $C_4$ -Kohlenwasserstoffrest, steht.

Nichtionische Tenside enthalten beipsielsweise als hydrophile Gruppe eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder $C_2$ - $C_6$ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- $C_{12}$-$C_{30}$-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol® - Typen (Cognis),
- alkoxilierte Triglyceride,
- alkoxilierte Fettsäurealkylester der Formel (Tnio-1)

$$R^1CO\text{-}(OCH_2CHR^2)_wOR^3 \qquad \text{(Tnio-1)}$$

  in der $R^1CO$ für einen linearen oder verzweigten, gesättigten und/oder ungesättigten Acylrest mit 6 bis 22 Kohlenstoffatomen, $R^2$ für Wasserstoff oder Methyl, $R^3$ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

$R^4O\text{-}[G]_p$ (Tnio-2)

in der $R^4$ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (Tnio-2) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest $R^4$ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge $C_8$-$C_{10}$ (DP = 1 bis 3), die als Vorlauf bei der destillativen Auftrennung von technischem $C_8$-$C_{18}$-Kokosfettalkohol anfallen und mit einem Anteil von weniger als 6 Gew.-% $C_{12}$-Alkohol verunreinigt sein können sowie Alkyloligoglucoside auf Basis technischer $C_{9/11}$-Oxoalkohole (DP = 1 bis 3). Der Alkyl- bzw. Alkenylrest $R^{15}$ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem $C_{12/14}$-Kokosalkohol mit einem DP von 1 bis 3.

- Zuckertenside vom Typ der Fettsäure-N-alkylpolyhydroxyalkylamide, ein nichtionisches Tensid der Formel (Tnio-3),

$R^5CO\text{-}NR^6\text{-}[Z]$ (Tnio-3)

in der $R^5CO$ für einen aliphatischen Acylrest mit 6 bis 22 Kohlenstoffatomen, $R^6$ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (Tnio-4) wiedergegeben werden:

$R^7CO\text{-}(NR^8)\text{-}CH_2\text{-}[CH(OH)]_4\text{-}CH_2OH$ (Tnio-4)

Vorzugsweise werden als Fettsäure-N-alkylpolyhydroxyalkylamide Glucamide der Formel (Tnio-4) eingesetzt, in der $R^8$ für Wasserstoff oder eine Alkylgruppe steht und $R^7CO$ für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (Tnio-4), die durch reduktive Aminierung von Glucose mit Methylamin und anschließende Acylierung mit Laurinsäure oder C12/14-Kokosfettsäure beziehungsweise einem entsprechenden Derivat erhalten werden. Weiterhin können sich die Polyhydroxyalkylamide auch von Maltose und Palatinose ableiten.

Weitere typische Beispiele für nichtionische Tenside sind Fettsäureamidpolyglycolether, Fettaminpolyglycolether, Mischether bzw. Mischformale, Proteinhydrolysate (insbesondere pflanzliche Produkte auf Weizenbasis) und Polysorbate.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure sowie die Zuckertenside erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Das bzw. die Tenside können - bezogen auf das Gesamtgewicht des Mittels - in einer Gesamtmenge von 0,1 bis 18,0 Gew.-%, bevorzugt von 0,5 bis 16,0 Gew.-%, weiter bevorzugt von 1,0 bis 14,0 Gew.-% und ganz besonders bevorzugt von 1,5 bis 12,0 Gew.-% im Mittel enhalten sein.

<u>weitere optionale Inhalttstoffe im Mittel</u>

Zusätzlich zu den bereits beschriebenen erfindungswesentlichen Bestandteilen (a1) bis (a3) kann das Mittel zusätzlich auch noch weitere optionale Inhaltsstoffe enthalten.

So können die Mittel ferner auch noch weitere Wirk-, Hilfs- und Zusatzstoffe enthalten, wie beispielsweise Lösungsmittel, Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, $N_2O$, Dimethylether, $CO_2$ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

<u>pH-Wert der Mittel</u>

Die pH-Werte des erfindungsgemäßen Mittels können auf einen leicht sauren bis alkalischen pH-Wert eingestellt werden. Ganz besonders bevorzugt besitzt das Färbemittel (F) einen pH-Wert im Bereich von 5,0 bis 10,0, bevorzugt von 6,0 bis 9,5, weiter bevorzugt von 6,0 bis 8,7 und ganz besonders bevorzugt von 6,0 bis 7,5.

Zur Einstellung der jeweiligen gewünschten pH-Werte können die dem Fachmann bekannten Alkalisierungsmittel und Acidifizierungsmittel verwendet werden. Bei den pH-Werten im Sinne der vorliegenden Erfindung handelt es sich um pH-Werte, die bei einer Temperatur von 22°C gemessen wurden.

Als Alkalisierungsmittel können die Mittel beispielsweise Ammoniak, Alkanolamine und/oder basische Aminosäuren enthalten.

Die in dem erfindungsgemäßen Mittel einsetzbaren Alkanolamine werden bevorzugt ausgewählt aus primären Aminen mit einem $C_2$-$C_6$-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Erfindungsgemäß besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das

erfindungsgemäße Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -$SO_3$H-Gruppe enthält. Bevorzugte Aminosäuren sind Aminocarbonsäuren, insbesondere $\alpha$-(alpha)-Aminocarbonsäuren und $\omega$-Aminocarbonsäuren, wobei $\alpha$-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische $\alpha$-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ebenfalls erfindungsgemäß ist die Einstellung des gewünschten pH-Werte über ein Puffersystem. Unter einem Puffer oder einem Puffersystem wird üblicherweise ein Gemisch aus einer schwachen oder mittelstarken Säure (z. B. Essigsäure) mit einem praktisch völlig dissoziierten Neutralsalz derselben Säure (z. B. Natriumacetat) verstanden. Wird etwas Base oder Säure zugegeben, so ändert sich der pH-Wert kaum (Pufferung). Die Wirkung der in einer Pufferlösung enthaltenen Puffersubstanzen beruht auf der Abfangreaktion von Wasserstoff- bzw. Hydroxid-Ionen unter Bildung schwacher Säuren bzw. Basen aufgrund ihres Dissoziationsgleichgewichts.

Ein Puffersystem kann aus einem Gemisch einer anorganischen oder organischen Säure und einem korrespondierenden Salz dieser Säure ausgebildet werden.

Säuren können gepuffert werden durch alle Salze aus schwachen Säuren und starken Basen, Basen durch Salze aus starken Säuren und schwachen Basen. Die starke (vollständig in Ionen dissoziierte) Salzsäure kann z. B. durch Zusatz von Natriumacetat abgepuffert werden. Entsprechend dem Gleichgewicht

$$H_3C{-}COONa + HCl \rightleftharpoons NaCl + H_3C{-}COOH$$

wird Salzsäure durch Natriumacetat unter Bildung von Kochsalz in die schwache Essigsäure übergeführt, die in Gegenwart eines Natriumacetat-Überschusses nur zu einem sehr geringen Anteil dissoziiert. Puffer, die sowohl gegenüber Säuren als auch Basen wirken, sind Gemische aus schwachen Säuren und ihren Salzen.

Literaturbekannte Beispiele für Puffersysteme sind Essigsäure/Natriumacetat, Borsäure/Natriumborat, Phosphorsäure/Natriumphosphat und Hydrogencarbonat/Soda.

Der pH-Wert des erfindungsgemäßen Mittels kann zum Beispiel durch Zusatz eines anorganischen oder organischen Puffersystems eingestellt werden. Unter einem anorganischen Puffersystem wird im Sinne der vorliegenden Erfindung eine Mischung aus einer anorganischen Säure und ihrer konjugierten korrespondieren anorganischen Base verstanden.

Unter einem organischen Puffersystem wird im Sinne der vorliegenden Erfindung eine Mischung aus einer organischen Säure und ihrer konjugierten korrespondieren Base verstanden. Bedingt durch den organischen Säure-Rest ist auch die konjugierte korrespondierende Base der organischen Säure ebenfalls organisch. Hierbei kann das zur Neutralisation der Ladung des Säure-Anions vorhandene Kation anorganisch oder organisch sein.

Beispiele für anorganische Säuren sind Schwefelsäure, Salzsäure und Phosphorsäure ($H_3PO_4$). Bei Phosphorsäure handelt es sich um eine mittelstarke Säure, die ganz besonders bevorzugt ist.

Eine ganz besonders gut geeignete anorganische Säure ist Kaliumdihydrogenphosphat Kaliumdihydrogenphosphat besitzt die Summenformel $KH_2PO_4$ und trägt die CAS-Nummer 7778-77-0. Kaliumdihydrogenphosphat besitzt eine molare Masse von 136,09 g/mol. Es ist gut löslich in Wasser (222 g/l bei 20 °C) und reagiert in Wasser sauer. Eine 5 % ige Lösung von Kaliumdihydrogenphosphat in Wasser besitzt einen pH-Wert von 4,4.

Eine weitere ganz besonders gut geeignete anorganische Säure ist Natriumdihydrogenphosphat. Natriumdihydrogenphosphat besitzt die Summenformel $NaH_2PO_4$ und trägt die CAS-Nummern 7558-80-7 (Anhydrat), 10049-21-5 (Monohdrat) und 13472-35-0 (Dihydrat). Das wasserfreie Natriumdihydrogenphosphat besitzt eine molare Masse von 119,98 g/mol. Natriumdihydrogenphosphat reagiert in wässriger Lösung sauer.

Besonders bevorzugt als korrespondierendes Salz der vorgenannten beiden Säuren ist Dikaliumhydrogenphosphat. Dikaliumhydrogenphosphat besitzt die Summenformel $K_2HPO_4$ und trägt die CAS-Nummern 7758-11-4 (wasserfrei) und 16788-57-1 (Trihydrat). Das wasserfreie Dikaliumhydrogenphosphat besitzt eine molare Masse von 174,18 g/mol. Dikaliumhydrogenphosphat reagiert in wässriger Lösung alkalisch.

Ebenfalls besonders bevorzugt als korresprondierdens Salz der vorgenannten beiden Säuren ist Dinatriumhydrogenphosphat. Dinatriumhydrogenphosphat besitzt die Summenformel $Na_2HPO_4$ und trägt die CAS-Nummern 7558-79-4 (wasserfrei), 10028-24-7 (Dihydrat), 7782-85-6 (Heptahydrat) und 10039-32-4 (Dodecahydrat). Das wasserfreie Dinatriumhydrogenphosphat besitzt eine molare Masse von 141,96 g/mol. Dinatriumhydrogenphosphat reagiert in wässriger Lösung alkalisch.

Beispiele für organische Säuren sind Zitronensäure, Bernsteinsäure, Weinsäure, Milchsäure, Essigsäure, Äpfelsäure, Malonsäure und Maleinsäure.

Beispiele für die korrespondierenden Salze dieser organischen Säuren sind die Natrium- und Kaliumsalze der Zitronensäure, die Natrium- und Kaliumsalze der Bernsteinsäure, die Natrium- und Kaliumsalze der Weinsäure, die Natrium- und Kaliumsalze der Milchsäure, die Natrium- und Kaliumsalze der Essigsäure, die Natrium- und Kaliumsalze der Äpfelsäure, die Natrium- und Kaliumsalze der Malonsäure und die die Natrium- und Kaliumsalze der Maleinsäure

Verfahren zum Färben von Keratinmaterial

Die zuvor beschriebenen Mittel lassen sich hervorragend in Verfahren zum Färben von keratinischem Material, insbesondere von menschlichen Haaren einsetzen.

Ein zweiter Gegenstand der vorliegenden Erfindung ist daher ein Verfahren zum Färben von keratinischem Material, insbesondere menschlichen Haaren, umfassend die folgenden Schritte:

(1) Anwendung eines Färbemittels auf dem keratinischem Material, wobei das Färbemittel ein Mittel ist, wie es bei der Beschreibung der ersten Erfindungsgegenstand im Detail offenbart wurde,
(2) Einwirken des Färbemittels auf dem keratinischen Material und
(3) Ausspülen des Färbemittels mit Wasser.

In Schritt (1) des erfindungsgemäßen Verfahrens wird das Mittel des ersten Erfindungsgegenstand auf dem keratinische Material, bei dem es sich ganz besonders bevorzugt um menschliche Haare handelt, angewendet.

In Schritt (2) des erfindungsgemäßen Verfahrens wird das Mittel dann nach seiner Applikation auf das keratinische Material einwirken gelassen. In diesem Zusammenhang sind verschiedene Einwirkzeiten von beispielsweise 30 Sekunden bis 60 Minuten denkbar.

Ein großer Vorteil des erfindungsgemäßen Färbesystems liegt jedoch darin, dass auch in sehr kurzen Zeiträumen nach kurzen Einwirkzeiten ein intensive Farbergebnis erzielt werden kann. Aus diesem Grund ist es von Vorteil, wenn die Anwendungsmischung nach ihrer Applikation nur für vergleichsweise kurze Zeiträume von 30 Sekunden bis 15 Minuten, bevorzugt von 30 Sekunden bis 10 Minuten, und besonders bevorzugt von 1 bis 5 Minuten auf dem Keratinmaterial verbleibt.

In einer weiteren bevorzugten Ausführungsform ist ein erfindungsgemäßes Verfahren gekennzeichnet durch das

(2) Einwirken des Färbemittels auf dem keratinischen Material für einen Zeitraum von 30 Sekunden bis 15 Minuten, bevorzugt von 30 Sekunden bis 10 Minuten, und besonders bevorzugt von 1 bis 5 Minuten.

Im Anschluss an das Einwirken der Anwendungsmischung auf das Keratinmaterial wird diese schließlich in Schritt (3) des Verfahrens mit Wasser ausgespült.

Hierbei kann die Awendungsmischung in einer Ausführungsform nur mit Wasser, d.h. ohne Zuhilfenahme eines Nachbehandlungsmittels oder eines Shampoos, ausgewaschen werden. Auch die Anwendung eines Nachbehandlungsmittels oder Conditioners in Schritt (6) ist prinzipiell denkbar.

Zur Lösung der erfindungsgemäßen Aufgabenstellung und zur Erhöhung des Anwendungskomforts hat es sich jedoch als ganz besonders bevorzugt herausgestellt, das Ausspülen des Mittels in Schritt (3) ausschließlich mit Wasser ohne Zuhilfenahme eines weiteren Nachbehandlungsmittels, Shampoos oder Conditioners vorzunehmen.

In einer weiteren bevorzugten Ausführungsform ist ein erfindungsgemäßes Verfahren gekennzeichnet durch das

(3) Ausspülen des Färbemittels ausschließlich mit Wasser.

Betreffend die weiteren bevorzugten Ausführungsformen der erfindungsgemäßen Verfahrens gilt *mutatis mutantis* das zum erfindungsgemäßen Mittel gesagte.

Beispiele

1. Formulierungen

Es wurden die folgenden Formulierungen hergestellt (alle Angaben, sofern nichts anderes angegeben ist, in Gew.-%):

| Färbemittel | (V1) | (V2) | (E1) | (E2) |
|---|---|---|---|---|
| Cetylalkohol | 3,0 | 3,0 | 3,0 | 3,0 |
| $C_{12}$-$C_{18}$-Fettalkohole (Lorol techn.) | 3,0 | 3,0 | 3,0 | 3,0 |
| Phenoxyethanol | 0,9 | 0,9 | 0,9 | 0,9 |
| Natriumsalicylat | 0,4 | 0,4 | 0,4 | 0,4 |
| Unipure Red LC 3071 (CI 15850) | 1,0 | 1,0 | 1,0 | 1,0 |
| Dow Corning 2-8566 (Siloxanes and Silicones, 3-[(2-Aminoethyl)amino]-2-methylpropyl Me, Di-Me-Siloxane | 1,0 | --- | 1,0 | 1,0 |
| Belsil ADM 8301 E (Amodimethicone/Morpholinomethyl Silsesquioxane Copolymer, Wacker) | --- | 1,0 | --- | --- |
| 1,2-Propandiol | 10,0 | 10,0 | 10,0 | 10,0 |
| Kaliumdihydrogenphosphat | 0,35 | 0,35 | 0,35 | 0,35 |
| Dinatriumhydrogenphosphat | 0,72 | 0,72 | 0,72 | 0,72 |
| Emuglade CM (BASF, Cetearyl Isononanoat, Ceteareth-20, Cetearylalcohol, Glycerylsteareat, Glycerin, Ceteareth-12, Cetylpalmitat) | 3,0 | 3,0 | 3,0 | 3,0 |
| Natrosol 250 HR (Hydroxyethylcellulose) | --- | --- | 2,0 | --- |
| Keltrol CG-SFT (CP, Kelco, Xanthan Gum) | --- | 2,0 | --- | 2,0 |
| Wasser | ad 100 | ad 100 | ad 100 | ad 100 |

2. Anwendung

Die zuvor hergestellten Mittel wurden auf Haarsträhnen (Kerling, Euronaturhaar weiß Flottenverhältnis: 1 g Färbemittel (E1) pro g Haarsträhne) appliziert. Das Mittel wurde für fünf Minuten einwirken gelassen. Im Anschluss daran wurden die Haarsträhne gründlich (1 Minute) mit Wasser ausgewaschen, getrocknet und dann farbmetrisch mit einem Farbmessgerät der Firma Datacolor, Typ Spectraflash 450 vermessen.

Der für die Beurteilung der unterschiedlichen Farbintensitäten herangezogene dE-Wert ergibt sich aus den am jeweiligen Strähnenteil gemessenen L*a*b*-Farbmesswerten wie folgt:

$$dE = [\ (L_i - L_0)^2 + (a_i - a_0)^2 + (b_i - b_0)]^{1/2}$$

$L_0$, $a_0$ und $b_0$ = Messwerte der uncolorierten Strähne
$L_i$, $a_i$ und $b_i$ = Messwerte der mit gefärbten Strähne

Je größer der dE-Wert ist, desto höher ist der Farbabstand zwischen ungefärbter und gefärbter Strähen und desto höher ist die Farbintensität.

| Mittel | L | a | b | dE |
|---|---|---|---|---|
| Kerling, uncoloriert | 73,18 | 2,35 | 22,09 | -- |
| Vergleich (V1) | 44,17 | 45,43 | 7,40 | 53,97 |
| Vergleich (V2) | 65,32 | 11,00 | 8,50 | 17,92 |
| Erfindung (E1) | 38,11 | 45,76 | 9,03 | 57,31 |
| Erfindung (E2) | 39,98 | 46,67 | 9,39 | 56,81 |

**Patentansprüche**

1. Mittel zum Färben von keratinischem Material, insbesondere menschlichen Haaren, enthaltend

   (a1) mindestens ein lineares, aminofunktionalisiertes Silikonpolymer, und
   (a2) mindestens ein Pigment mit einer Löslichkeit in Wasser bei 25 °C von weniger als 0,05 g/L, und
   (a3) mindestens ein Polysaccharid, das ausgewählt ist aus der Gruppe aus Hydroxy-$C_1$-$C_{30}$-alkylcellulosen, Hydroxy-$C_1$-$C_{30}$-alkylmethylcellulosen, Hydroxy-$C_1$-$C_{30}$-alkylethyl-cellulosen, $C_1$-$C_{30}$-Alkylcellulosen, $C_6$-$C_{30}$-Alkylhydroxyethylcellulosen, Cellulose, Stärke, Xanthan, Carboxyalkylcellulose, Alginsäure, Carrageen, Tragant, Karaya-Gummi, Gummi Arabicum, Gellan, Pektin, Agaropektin und/oder deren Salzen,
   **dadurch gekennzeichnet, dass** das Mittel frei ist von Silikonharzen.

2. Mittel nach Anspruch 1, **dadurch gekennzeichnet, dass** es mindestens ein lineares, aminofunktionalisiertes Silikonpolymer (a1) mit mindestens einer sekundären Aminogruppe enthält.

3. Mittel nach einem der Ansprüche 1 bis 2 **dadurch gekennzeichnet, dass** es mindestens ein lineares, aminofunktionalisiertes Silikonpolymer (a1) enthält, das mindestens eine Struktureinheit der Formel (Si-Amino) umfasst,

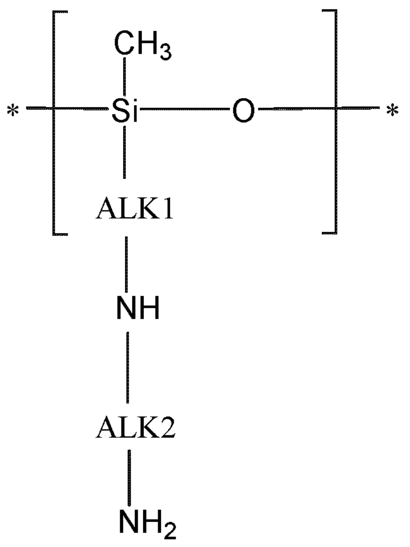

(Si-Amino)

   wobei

   ALK1 und ALK2 unabhängig voneinander für eine lineare oder verzweigte, zweiwertige $C_1$-$C_{20}$-Alkylengruppe stehen.

4. Mittel nach einem der Ansprüche 1 bis 3, **dadurch gekennzeichnet, dass** es mindestens ein lineares, aminofunktionalisiertes Silikonpolymer (a1) enthält, das Struktureinheiten der Formel (Si-I) und der Formel (Si-II) umfasst

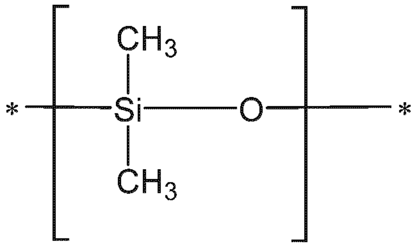
(Si-I)

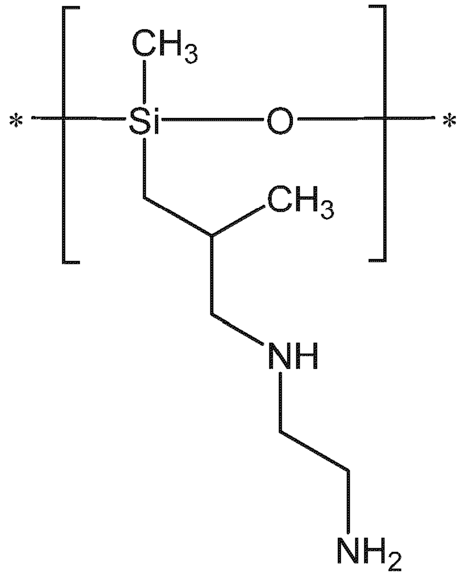
(Si-II).

5. Mittel nach einem der Ansprüche 1 bis 4, **dadurch gekennzeichnet, dass** es - bezogen auf das Gesamtgewicht des Mittels - ein oder mehrere lineare, aminofunktionalisierte Silikonpolymere (a1) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

6. Mittel nach einem der Ansprüche 1 bis 5, **dadurch gekennzeichnet, dass** es mindestens ein anorganisches Pigment (a2) enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

7. Mittel nach einem der Ansprüche 1 bis 6, **dadurch gekennzeichnet, dass** es - bezogen auf das Gesamtgewicht des Mittels - ein oder mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

8. Mittel nach einem der Ansprüche 1 bis 7, **dadurch gekennzeichnet, dass** es - bezogen auf das Gesamtgewicht des Mittels - ein oder mehrere Polysaccharide (a3), in einer Gesamtmenge von 0,1 bis 6,0 Gew.-%, bevorzugt 0,5 bis 5,0 Gew.-%, weiter bevorzugt von 1,0 bis 4,0 Gew.-% und ganz besonders bevorzugt von 1,5 bis 3,5 Gew.-% enthält.

9. Mittel nach einem der Ansprüche 1 bis 8, **dadurch gekennzeichnet, dass** es einen oder mehrere Fettbestandteile (a4) aus der Gruppe der $C_{12}$-$C_{30}$-Fettalkohole, der $C_{12}$-$C_{30}$-Fettsäuretriglyceride, der $C_{12}$-$C_{30}$-Fettsäuremonoglyceride, der $C_{12}$-$C_{30}$-Fettsäurediglyceride und/oder der Kohlenwasserstoffe enthält.

10. Verfahren zum Färben von keratinischem Material, insbesondere menschlichen Haaren, umfassend die folgenden Schritte:

    (1) Anwendung eines Färbemittels nach einem der Ansprüche 1 bis 9 auf dem keratinischem Material,
    (2) Einwirken des Färbemittels auf dem keratinischen Material und
    (3) Ausspülen des Färbemittels mit Wasser.

11. Verfahren nach Anspruch 10, **gekennzeichnet durch** das
    (2) Einwirken des Färbemittels auf dem keratinischen Material für einen Zeitraum von 30 Sekunden bis 15 Minuten, bevorzugt von 30 Sekunden bis 10 Minuten, und besonders bevorzugt von 1 bis 5 Minuten.

**Claims**

1. An agent for dyeing keratinous material, in particular human hair, containing

(a1) at least one linear, amino-functionalized silicone polymer, and
(a2) at least one pigment having a solubility in water at 25 °C of less than 0.05 g/L, and
(a3) at least one polysaccharide selected from the group of hydroxy $C_1$-$C_{30}$ alkylcelluloses, hydroxy $C_1$-$C_{30}$ alkylmethylcelluloses, hydroxy $C_1$-$C_{30}$ alkylethylcelluloses, $C_1$-$C_{30}$ alkylcelluloses, $C_6$-$C_{30}$ alkylhydroxyethylcelluloses, cellulose, starch, xanthan gum, carboxyalkylcellulose, alginic acid, carrageenan, tragacanth, karaya gum, gum arabic, gellan, pectin, agaropectin and/or the salts thereof,
**characterized in that** the agent is free of silicone resins.

2. The agent according to claim 1, **characterized in that** it contains at least one linear, amino-functionalized silicone polymer (a1) having at least one secondary amino group.

3. The agent according to one of claims 1 to 2, **characterized in that** it contains at least one linear, amino-functionalized silicone polymer (a1) which comprises at least one structural unit of the formula (Si-amino),

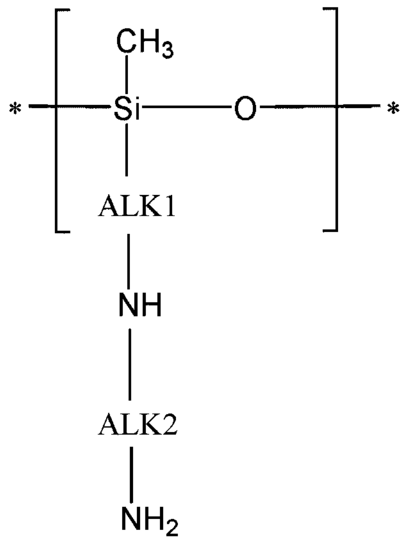

(Si-amino)

where
ALK1 and ALK2 represent, independently of one another, a linear or branched, divalent $C_1$-$C_{20}$ alkylene group.

4. The agent according to one of claims 1 to 3, **characterized in that** it contains at least one linear, amino-functionalized silicone polymer (a1) which comprises structural units of formula (Si-I) and formula (Si-II)

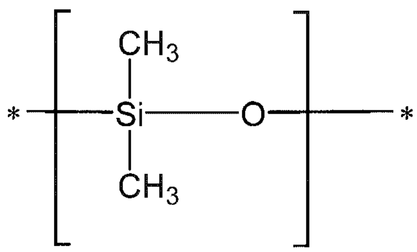

(Si-I)

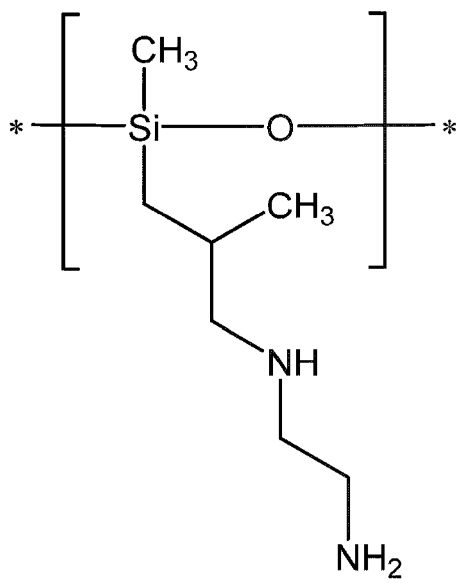

(Si-II).

5. The agent according to one of claims 1 to 4, **characterized in that** it contains, based on the total weight of the agent, one or more linear, amino-functionalized silicone polymers (a1) in a total amount from 0.1 to 8.0 wt.%, preferably from 0.2 to 5.0 wt.%, more preferably from 0.3 to 3.0 wt.%, and very particularly preferably from 0.4 to 2.5 wt.%.

6. The agent according to one of claims 1 to 5, **characterized in that** it contains at least one inorganic pigment (a2), which is preferably selected from the group of colored metal oxides, metal hydroxides, metal oxide hydrates, silicates, metal sulfides, complex metal cyanides, metal sulfates, bronze pigments, and/or from mica-based colored pigments which are coated with at least one metal oxide and/or a metal oxychloride.

7. The agent according to one of claims 1 to 6, **characterized in that** it contains, based on the total weight of the agent, one or more pigments (a2) in a total amount from 0.01 to 10.0 wt.%, preferably from 0.1 to 5.0 wt.%, more preferably from 0.2 to 2.5 wt.%, and very particularly preferably from 0.25 to 1.5 wt.%.

8. The agent according to one of claims 1 to 7, **characterized in that** it contains, based on the total weight of the agent, one or more polysaccharides (a3) in a total amount from 0.1 to 6.0 wt.%, preferably from 0.5 to 5.0 wt.%, more preferably from 1.0 to 4.0 wt.%, and very particularly preferably from 1.5 to 3.5 wt.%.

9. The agent according to one of claims 1 to 8, **characterized in that** it contains one or more fatty components (a4) from the group of $C_{12}$-$C_{30}$ fatty alcohols, $C_{12}$-$C_{30}$ fatty acid triglycerides, $C_{12}$-$C_{30}$ fatty acid monoglycerides, $C_{12}$-$C_{30}$ fatty acid diglycerides and/or hydrocarbons.

10. A method for dyeing keratinous material, in particular human hair, comprising the following steps:

    (1) applying a dyeing agent according to one of claims 1 to 9 to the keratinous material,
    (2) allowing the dyeing agent to act on the keratinous material, and
    (3) rinsing out the dyeing agent using water.

11. The method according to claim 10, **characterized by**
    (2) allowing the dyeing agent to act on the keratinous material for a period of 30 seconds to 15 minutes, preferably 30 seconds to 10 minutes, and particularly preferably 1 to 5 minutes.

## Revendications

1. Agent permettant la coloration de matière kératinique, en particulier de cheveux humains, contenant (a1) au moins un polymère de silicone linéaire aminofonctionnalisé, et

    (a2) au moins un pigment comportant une solubilité dans l'eau à 25 °C inférieure à 0,05 g/l, et
    (a3) au moins un polysaccharide qui est choisi dans le groupe constitué d'hydroxy-alkyl en $C_1$-$C_{30}$-celluloses, hydroxy-alkyl en $C_1$-$C_{30}$-méthylcelluloses, hydroxy-alkyl en $C_1$-$C_{30}$-éthyl-celluloses, alkyl en $C_1$-$C_{30}$-celluloses, alkyl en $C_6$-$C_{30}$-hydroxy-éthyl-celluloses, cellulose, amidon, gomme xanthane, carboxyalkylcellulose, acide alginique, carraghénane, gomme adragante, gomme karaya, gomme arabique, gomme gellane, pectine, agaropectine et/ou leurs sels,
    **caractérisé en ce que** l'agent est exempt de résines de silicone.

2. Agent selon la revendication 1, **caractérisé en ce qu'il** contient au moins un polymère de silicone linéaire aminofonctionnalisé (a1) comportant au moins un groupe amine secondaire.

3. Agent selon l'une des revendications 1 à 2, **caractérisé en ce qu'**il contient au moins un polymère de silicone linéaire aminofonctionnalisé (a1) qui comprend au moins un motif structural de formule (Si-Amino),

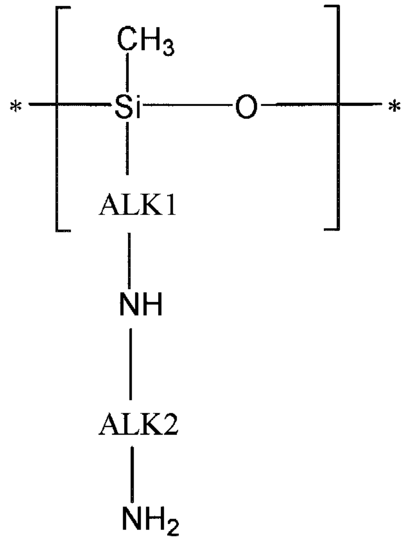

(Si-Amino)

où

ALK1 et ALK2 représentent, indépendamment l'un de l'autre, un groupe alkylène en $C_1$-$C_{20}$ bivalent, linéaire ou ramifié.

**4.** Agent selon l'une des revendications 1 à 3, **caractérisé en ce qu'il** contient au moins un polymère de silicone linéaire aminofonctionnalisé (a1) qui comprend des motifs structuraux de formule (Si-I) et de formule (Si-II)

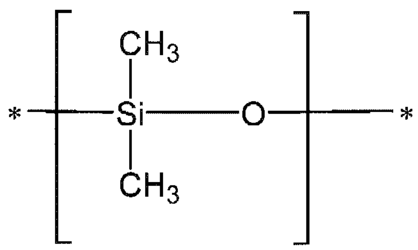

(Si-I)

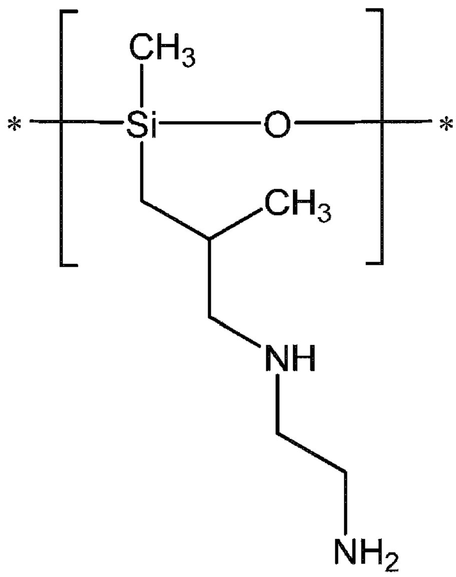

(Si-II).

**5.** Agent selon l'une des revendications 1 à 4, **caractérisé en ce qu'il** contient, par rapport au poids total de l'agent, un ou plusieurs polymères de silicone linéaires aminofonctionnalisés (a1) en une quantité totale allant de 0,1 à 8,0 % en poids, de préférence de 0,2 à 5,0 % en poids, plus préférablement de 0,3 à 3,0 % en poids et de manière tout particulièrement préférée de 0,4 à 2,5 % en poids.

**6.** Agent selon l'une des revendications 1 à 5, **caractérisé en ce qu'**il contient au moins un pigment inorganique (a2) qui est de préférence choisi dans le groupe constitué d'oxydes métalliques colorés, hydroxydes métalliques colorés, oxydes métalliques hydratés colorés, silicates colorés, sulfures métalliques colorés, cyanures métalliques complexes colorés, sulfates métalliques colorés, pigments de bronze colorés et/ou de pigments colorés à base de mica qui sont recouverts d'au moins un oxyde métallique et/ou d'un oxychlorure métallique.

**7.** Agent selon l'une des revendications 1 à 6, **caractérisé en ce qu'il** contient, par rapport au poids total de l'agent, un ou

plusieurs pigments (a2) en une quantité totale allant de 0,01 à 10,0 % en poids, de préférence de 0,1 à 5,0 % en poids, plus préférablement de 0,2 à 2,5 % en poids et de manière tout particulièrement préférée de 0,25 à 1,5 % en poids.

8. Agent selon l'une des revendications 1 à 7, **caractérisé en ce qu'il** contient, par rapport au poids total de l'agent, un ou plusieurs polysaccharides (a3), en une quantité totale allant de 0,1 à 6,0 % en poids, de préférence de 0,5 à 5,0 % en poids, plus préférablement de 1,0 à 4,0 % en poids et de manière tout particulièrement préférée de 1,5 à 3,5 % en poids.

9. Agent selon l'une des revendications 1 à 8, **caractérisé en ce qu'**il contient un ou plusieurs constituants gras (a4) choisis dans le groupe des alcools gras en $C_{12}$-$C_{30}$, triglycérides d'acides gras en $C_{12}$-$C_{30}$, monoglycérides d'acides gras en $C_{12}$-$C_{30}$, diglycérides d'acides gras en $C_{12}$-$C_{30}$ et/ou hydrocarbures.

10. Procédé permettant la coloration de matière kératinique, en particulier de cheveux humains, comprenant les étapes suivantes :

    (1) application d'un agent de coloration selon l'une des revendications 1 à 9 sur la matière kératinique,
    (2) action de l'agent de coloration sur la matière kératinique, et
    (3) élimination par rinçage de l'agent de coloration avec de l'eau.

11. Procédé selon la revendication 10, **caractérisé par**
(2) l'action de l'agent de coloration (a) sur la matière kératinique pendant une durée allant de 30 secondes à 15 minutes, de préférence de 30 secondes à 10 minutes, et de manière particulièrement préférée de 1 à 5 minutes.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20110104094 A1
- US 2016051023 A1
- DE 102013226102 A1
- EP 3058935 B1
- DE 19738866 A


### In der Beschreibung aufgeführte Nicht-Patentliteratur


- *CHEMICAL ABSTRACTS*, 106842-44-8
- *CHEMICAL ABSTRACTS*, 7558-80-7
- *CHEMICAL ABSTRACTS*, 10049-21-5
- *CHEMICAL ABSTRACTS*, 13472-35-0
- *CHEMICAL ABSTRACTS*, 7758-11-4
- *CHEMICAL ABSTRACTS*, 16788-57-1
- *CHEMICAL ABSTRACTS*, 7558-79-4
- *CHEMICAL ABSTRACTS*, 10028-24-7
- *CHEMICAL ABSTRACTS*, 7782-85-6
- *CHEMICAL ABSTRACTS*, 10039-32-4